# EUROPEAN PATENT APPLICATION

(11) **EP 4 607 520 A1**
(43) Date of publication of application: **27.08.2025**
(21) Application number: 23880306.8
(22) Date of filing: 20.10.2023
(51) Int. Cl.: G16B 50/00, G16B 25/20, G16B 45/00

(54) **METHOD OF GUIDING MULTIPLE DETAILED PERFORMANCE EXPERIMENTS REQUIRED FOR DEVELOPING REAGENT FOR DETECTING TARGET NUCLEIC ACID MOLECULE, AND ORGANIZING EXPERIMENT RESULT RECORD SHEETS**

(30) Priority: 20.10.2022 KR 20220135902
(71) Applicant: Seegene, Inc., Seoul 05548 (KR)
(72) Inventor: LEE, Kwang Il, Seoul 05548 (KR); GU, Gyeong Mo, Seoul 05548 (KR); NOH, Hyo Jeong, Seoul 05548 (KR); OH, Myoung Suk, Seoul 05548 (KR); OH, Hye Jin, Seoul 05548 (KR); CHO, Seong Min, Seoul 05548 (KR); KIM, Han Deul, Seoul 05548 (KR); KIM, Ka In, Seoul 05548 (KR); KOO, Won Jun, Seoul 05548 (KR); KANG, Eun Ju, Seoul 05548 (KR); YU, Jin Yeong, Seoul 05548 (KR)
(74) Representative: Viering, Jentschura & Partner mbB Patent- und Rechtsanwälte
(86) International application number: PCT/KR2023/016418
(87) International publication number: WO 2024/085731

(57) **Abstract**

This method for guiding at least two detailed performance experiments the method comprising: providing a data input screen for at least one requirement item selected among items for experimentation to be used in performing each detailed performance experiment on an oligonucleotide for detecting the target nucleic acid molecule, wherein the items include materials, experimental procedures, experimental devices and a combination thereof; displaying preparation information for a reaction plate for performing, by a nucleic acid detection device, an amplification reaction using the oligonucleotide on the basis of the data input via the data input screen; receiving result data of the amplification reaction performed by the nucleic acid detection device by using an amplification composition containing the oligonucleotide, on the basis of the preparation information; processing/analyzing the received result data of the amplification reaction to obtain data; and storing the data input via the data input screen, the preparation information, the amplification reaction result, and the processed/analyzed data as an experimental result documentation for each detailed performance experiment.

## Description

### [Technical Field]

The present invention relates to the guidance of at least two detailed performance experiments required for the development of a detection reagent for a target nucleic acid molecule, the organization of experimental result documentation, and the management and storage of research and development data generated during the research and development of a detection reagent for a target nucleic acid molecule.

### [Background Art]

Molecular diagnostics is a rapidly growing field in the in vitro diagnostic market for early disease diagnosis. Among these methods, methods using nucleic acids are being effectively used to diagnose genetic causes of infections such as viruses and bacteria, based on high specificity and sensitivity.

Most nucleic acid-based diagnostic methods use nucleic acid amplification reactions to amplify a target nucleic acid (e.g., viral or bacterial nucleic acids). A representative example is the polymerase chain reaction (PCR), among a nucleic acid amplification reaction, which involves repeated cyclic processes of denaturation of double-stranded DNA, annealing of oligonucleotide primers to the DNA template, and primer extension by DNA polymerase (Mullis et al., U.S. Patents No. 4,683,195, 4,683,202, and 4,800,159; Saiki et al., Science 230:1350-1354, 1985). Other methods for nucleic acid amplification may include ligase chain reaction (LCR) (U.S. Patents No. 4,683,195 and 4,683,202; PCR Protocols: A Guide to Methods and Applications, Innis et al., eds., 1990), strand displacement amplification (SDA) (Walker et al., Nucleic Acids Res. 20(7):1691-6, 1992; Walker, PCR Methods Appl 3(1):1-6, 1993), transcription-mediated amplification (Phyffer et al., J. Clin. Microbiol. 34:834-841, 1996; Vuorinen et al., J. Clin. Microbiol. 33:1856-1859, 1995), nucleic acid sequence-based amplification (NASBA) (Compton, Nature 350(6313):91-92, 1991), rolling circle amplification (RCA) (Lisby, Mol. Biotechnol. 12(1):75-99, 1999; Hatchett et al., Genet. Anal. 15(2):35-40, 1999), Q-beta replicase (Lizardi et al., Biotechnology 6:1197, 1988), loop-mediated isothermal amplification (LAMP, Y. Mori, H. Kanda and T. Notomi, J. Infect. Chemother., 2013, 19, 404-411), recombinase polymerase amplification (RPA, J. Li, J. Macdonald and F. von Stetten, Analyst, 2018, 144, 31-67), and the like.

Recently, at least two diagnostic technologies have been used to detect at least two target nucleic acids within a single tube, based on these nucleic acid amplification reactions. For example, there are various at least two technologies to detect different types of viruses at once, using methods such as PCR and LAMP, which are examples of nucleic acid amplification reactions described above.

These nucleic acid amplification reaction technologies require the use of reagents for target nucleic acid detection, which include oligonucleotides (e.g., primers and/or probes) that specifically hybridize to the target nucleic acid, labels, DNA polymerase, dNTPs, Mg²⁺ ions, and buffers, in order to amplify and detect the target nucleic acid of interest.

Reagents for target nucleic acid detection are developed according to molecular diagnostic development protocols. During the development process, the selection of sophisticated oligonucleotides and various performance experiments for reagent development and commercialization are essential.

However, for those who are not experienced developers, conducting various performance experiments may not be easy.

Further, in each performance experiment, since tests are performed for each oligonucleotide to select an optimal one from at least two candidate oligonucleotides, a vast amount of research and development data accumulates, ranging from experiment planning, the materials used in the experiment, the experimental procedures based thereon, to the experimental results and their analysis.

This research and development data is derived from the development process to determine the optimal oligonucleotide for the detection reagent for a target nucleic acid molecule to be developed from among the candidate oligonucleotides. Therefore, the research and development data can be effectively used in the research and development process of the corresponding reagent, in the follow-up research process when subsequent actions are required after the corresponding reagent is sold, or in the research and development process of similar reagents.

### [Disclosure]

### [Technical Problem]

An object to be solved according to an embodiment is to provide a method of guiding at least two detailed performance experiments required for the development of a detection reagent for a target nucleic acid molecule, and storing the result data from each detailed performance experiment derived in this process in experimental result documentation.

An object to be solved according to an embodiment is to provide a method of managing the research and development data derived from the aforementioned research and development process.

Specifically, these objects may include providing a technology that organizes, stores, and allows for the retrieval of various research and development data derived from the research and development process, so that such data can be reused or referenced.

However, the problem to be solved by the present disclosure is not limited to that mentioned above, and other problems to be solved that are not mentioned may be clearly understood by those of ordinary skill in the art to which the present disclosure belongs from the following description.

### [Technical Solution]

In accordance with an aspect of a method for guiding at least two detailed performance experiments required for development of a detection reagent for a target nucleic acid molecule and organizing an experimental result documentation, to be performed by a computer device, the method comprising: providing a data input screen for at least one item for experimentation selected among items for experimentation to be used in performing each detailed performance experiment on an oligonucleotide for detecting the target nucleic acid molecule, wherein the items, include materials, experimental procedures, experimental devices, and a combination thereof; displaying preparation information for a reaction plate, to be used for a nucleic acid detection device to perform an amplification reaction using the oligonucleotide, based on data input via the data input screen for the item for experimentation for each detailed performance experiment; receiving result data of the amplification reaction performed by the nucleic acid detection device, using an amplification composition including the oligonucleotide prepared according to the preparation information for each detailed performance experiment,; processing/analyzing the received result data of the amplification reaction to obtain processed/analyzed data; and storing the data input via the data input screen for the item for experimentation, the preparation information, the result of the amplification reaction, and the processed/analyzed data as an experimental result documentation for each detailed performance experiment.

Also, the method may further comprise displaying a detailed performance experiment input screen for selecting types of the at least two detailed performance experiments, wherein each of the at least two detailed performance experiments may be for testing performance of the oligonucleotide, and the performance of the oligonucleotide may include sensitivity and/or specificity for the target nucleic acid molecule.

Also, wherein the at least two detailed performance experiments may be an experiments for selecting an oligonucleotide to be included in the detection reagent for the target nucleic acid molecule from at least two candidate oligonucleotides, and wherein the experiments for selecting may comprise: scoring the candidate oligonucleotides based on at least one selected among a group consisting of sensitivity, specificity, and amplification reaction efficiency of the at least two candidate oligonucleotides obtained from the received result data of the amplification reaction and/or the processed/analyzed data.

Also, wherein the sensitivity and specificity of the at least two candidate oligonucleotides may be obtained from the processed/analyzed data, and the amplification reaction efficiency of the at least two candidate oligonucleotides are obtained from the amplification reaction result.

Also, wherein the sensitivity, the specificity, and the amplification reaction efficiency of the at least two candidate oligonucleotides may be obtained from the processed/analyzed data.

Also, the method, may further comprise: providing a shared data input screen for at least one item for experimentation selected among items for experimentation to be used in performing the at least two detailed performance experiments, wherein the items include materials, experimental procedures, experimental devices and a combination thereof at least two detailed performance experiments,

wherein a shared data input via the shared data input screen may be provided according to a user's selection as an input data to the data input screen for the item for experimentation for each detailed performance.

Also, wherein at least one experimental materials-related data input in the shared data input screen is selected among a group consisting of type and concentration of an oligonucleotide, type and concentration of a target nucleic acid molecule, a type of enzyme, buffer, a type of a reaction vessel, type and concentration of positive control, type and concentration of negative control, type and concentration of internal control, type and amount in use of water, and a combination thereof.

Also, wherein at least one experimental procedures -related data input in the shared data input screen is selected among a group consisting of information on a target nucleic acid molecule for each detection channel, an amplification reaction protocol, an extraction method of the target nucleic acid molecule, and a combination thereof.

Also, wherein, at least one experimental procedures-related data input in the shared data input screen is selected among a group consisting of a nucleic acid detection device, an experimental preparation device, and a combination thereof.

Also, wherein the step of displaying the preparation information may comprise: displaying at least one selected from the group consisting of a type of a template and/or a concentration or series of concentrations of the template, a type of an oligonucleotide, and a type of a reaction plate, each of which is suitable for a purpose of the at least two detailed performance experiments.

Also, wherein the type of the template and/or the concentration or the series of concentrations of the template may be displayed in each of at least two reaction vessels included in the reaction plate.

Also, the method may further comprise: storing the data input via the data input screen as meta-data at a predetermined position within a pre-designed data storage structure; matching the received result data of the amplification reaction and the processed/analyzed data with the meta-data; and storing the result data of the amplification reaction and processed/analyzed data matched with the meta-data at a predetermined position within the data storage structure.

Also, the method may further comprise: providing a setup screen for setting up setting values used in processing/analyzing the result data of the amplification reaction; matching the setting values input via the setup screen with the meta-data; and storing the setting values matched with the meta-data at a predetermined position within the data storage structure.

Also, the method may further comprise: further comprising: providing a search condition input screen; receiving search conditions via the search condition input screen; searching for meta-data that matches the received search conditions from meta-data stored at a predetermined position within the data storage structure; and obtaining the searched meta-data and/or result data of the amplification reaction matched with the searched meta-data.

Also, wherein the processing/analyzing of the received result data of the amplification reaction to obtain the processed/analyzed data may be performed via a setup screen and a default screen, wherein the setup screen sets up setting values used for processing/analysis in at least one of the at least two detailed performance experiments, and displays results where the setting values applied to, wherein in at least one of detailed performance experiments, the default screen displays results where setting values per-set up via the setup screen are applied to and wherein the setup screen and the default screen may be displayed on a single screen.

Also, wherein the storing as the experimental result documentation for each detailed performance experiment may comprise: collecting predefined results for each step; and organizing the collected predefined results according to a format predesignated for each detailed performance experiment.

Also, wherein the experimental result documentation may be an electronic document that may be, based on predefined criteria in at least one detailed performance experiment of the at least two detailed performance experiments, derived and recorded results required for the detailed performance experiment from the collected predefined results.

Also, wherein the at least two detailed performance experiments may be performance optimization experiments to optimize performance of an oligonucleotide selected from at least two candidate oligonucleotides as an oligonucleotide to be included in the reagent for detecting the target nucleic acid molecule, and wherein the at least two detailed performance experiments may include at least one of: sensitivity or specificity in case that an amplification composition including the selected oligonucleotide is used to detect target nucleic acid molecules of different concentrations; sensitivity and specificity for either a standard strain that needs to be detected by a amplification composition including the selected oligonucleotide or an excluded strain that needs to be excluded from detection; sensitivity and specificity in case that a amplification composition including the selected oligonucleotide is accommodated in different type of accommodation vessels and used for detection;

sensitivity and specificity in case that only a single target nucleic acid molecule is included, or at least two or more target nucleic acid molecules are accommodated together in a reaction vessel in which a amplification composition including the selected oligonucleotide is accommodated, wherein the amplification composition including the selected oligonucleotide is used for analyzing at least two target nucleic acid molecules, specificity for either clinical samples including a target nucleic acid molecule to be detected by a amplification composition including the selected oligonucleotide or clinical samples not including a target nucleic acid molecule.

Also, wherein the step of displaying the detailed performance experiment input screen for selecting types of the at least two detailed performance experiments may comprise: providing a section that displays a progress status as an icon for each of the items for experimentation for each detailed performance experiment, the preparation information, the result data of the amplification reaction, the processed/analyzed data, and the experimental result documentation wherein, selection the icon displays a screen showing detailed information of the corresponding the item for experimentation, the preparation information, the result data of the amplification reaction, the processed/analyzed data, or the experimental result documentation.

Also, the method may further comprise: providing a parameter input screen for inputting values of parameters for each manufacturing task, the values of parameters being required for driving an experimental preparation device preparing the amplification composition used for each of the detailed performance experiments, wherein the manufacturing tasks include at least one of: a detailed task for a template dilution used in an amplification reaction; a detailed task for an oligonucleotide mixture; a detailed task for a master mixture including enzyme, buffer, and the oligonucleotide mixture; and a detailed task for setting up an amplification reaction solution by dispensing the master mixture and the template dilution into a reaction vessel.

### [Advantageous Effects]

According to an embodiment, by guiding at least two detailed performance experiments required for the development of a detection reagent for a target nucleic acid molecule on a computer device, experiments can be easily conducted, and a normalized experimental result documentation for each detailed performance experiment can be generated without human error.

According to another embodiment, an interface for inputting research and development data or meta-data is provided. This allows, first, research and development data or meta-data to be stored without omission.

Second, the aforementioned research and development data or meta-data can be stored in a structured format, that is, normalized. These data can be reused to improve or modify the corresponding research and development, or for another research and development. In this case, for smooth reuse, these data need to be stored according to a predefined standard. In this regard, in an embodiment, during the input process of these data or the process of storing these data, the data can be stored in a structured format, that is, in a normalized state.

Third, the aforementioned research and development data or meta-data can be easily searched. Specifically, in an embodiment, the search for the aforementioned research and development data or meta-data can be performed according to the conditions desired by the researcher or developer.

### [Description of Drawings]

FIG. 1 illustrates a computer device according to an embodiment, along with a nucleic acid detection device connected thereto and database.
FIG. 2 is a block diagram of the computer device according to an embodiment.
FIG. 3 is a shared data input screen provided on the display unit of the computer device according to an embodiment and FIG. 4 is another input screen provided on the display unit of the computer device according to an embodiment.
FIG. 5 exemplarily illustrates detailed performance experiments according to an embodiment.
FIG. 6 exemplarily illustrates an embodiment of screens provided for requirement items for performing an experiment for each detailed performance experiment (Material & Method) according to an embodiment.
FIG. 7 exemplarily illustrates plate information generated based on Material & Method according to an embodiment of the computer device.
FIG. 8 is an exemplified view illustrating the form of the experimental result documentation (lab note) generated in the computer device according to an embodiment.
FIG. 9 is an exemplified view illustrating the Setting value display screen according to an embodiment of the computer device.
FIG. 10 is a flowchart illustrating a method of managing research and development data in the research and development data management device according to an embodiment of the computer device.
FIG. 11 is a flowchart illustrating a method of guiding detailed performance experiments and organizing experimental result documentation according to an embodiment of the computer device.

### [Mode for Disclosure]

The advantages and features of the embodiments and the methods of accomplishing the embodiments will be clearly understood from the following description taken in conjunction with the accompanying drawings. However, embodiments are not limited to those embodiments described, as embodiments may be implemented in various forms. It should be noted that the present embodiments are provided to make a full disclosure and also to allow those skilled in the art to know the full range of the embodiments. Therefore, the embodiments are to be defined only by the scope of the appended claims.

In describing the embodiments of the present invention, detailed descriptions of well-known functions or configurations will be omitted if it is determined that they may unnecessarily obscure the gist of the present invention. In addition, in certain cases, there are terms arbitrarily selected by the applicant, and in this case, the meaning of the terms will be described in detail in the description of the corresponding invention. Therefore, the terms used in the present disclosure should be defined based on the meaning of the terms and the overall contents of the present disclosure, not just the name of the terms.

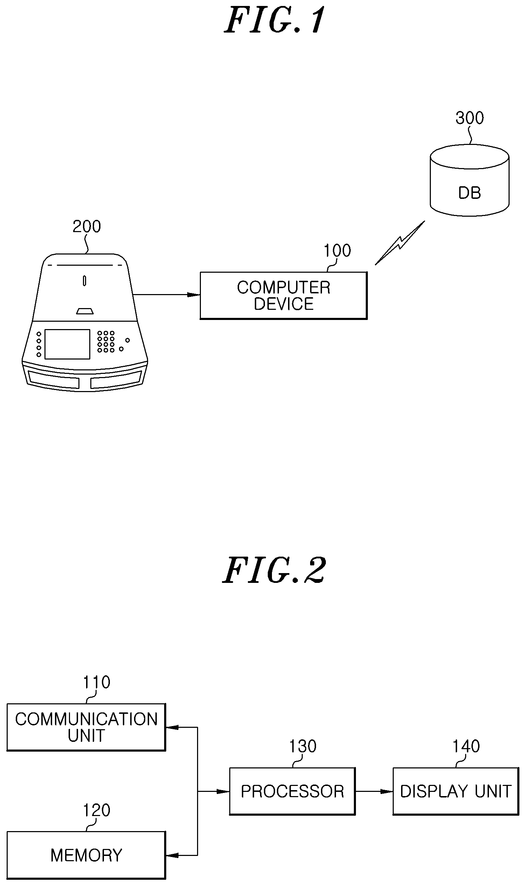

Before describing FIG. 1, the terms used in the present specification will be examined.

The term "target analyte" encompasses various substances (e.g., biological and non-biological substances), and it may refer to the same object as the term "target analyzed substance."

Such target analytes may specifically include biological substances, more specifically include at least one of nucleic acid molecules (e.g., DNA and RNA), proteins, peptides, carbohydrates, lipids, amino acids, biological compounds, hormones, antibodies, antigens, metabolites, and cells.

The term "sample" refers to both biological samples (e.g., cells, tissues, and bodily fluids) and non-biological samples (e.g., food, water, and soil). Among these, the biological sample may include, for example, at least one of virus, bacteria, tissue, cells, blood (including whole blood, plasma, and serum), lymph, bone marrow fluid, saliva, sputum, swab, aspiration, milk, urine, stool, ocular fluid, semen, brain extracts, cerebrospinal fluid, synovial fluid, thymic fluid, bronchoalveolar lavage fluid, ascitic fluid, and amniotic fluid. These samples may or may not include the aforementioned target analyzed substances.

Meanwhile, when the aforementioned target analyzed substance is a nucleic acid molecule or includes a nucleic acid molecule, the sample, which is presumed to include the target analyzed substance, may undergo a nucleic acid extraction process known in the art (refer to: Sambrook, J. et al., Molecular Cloning: A Laboratory Manual, 3rd ed., Cold Spring Harbor Press, 2001). The nucleic acid extraction process may vary depending on the type of sample. Additionally, when the extracted nucleic acid is RNA, a reverse transcription process may be undergone to synthesize cDNA (refer to: Sambrook, J. et al., Molecular Cloning: A Laboratory Manual, 3rd ed., Cold Spring Harbor Press, 2001).

The term "data set" refers to the data obtained from a signal generation reaction for the target analyzed substance using a signal generation means (which will be described below).

In this case, the term "signal generation reaction" refers to a reaction that generates a signal depending on the properties of the target analyzed substance in the sample, such as activity, quantity, or presence (or absence), specifically organizing a signal depending on the presence (or absence). Such signal generation reactions include both biological reactions and chemical reactions. Among these, biological reactions include genetic analysis processes such as PCR, real-time PCR, isothermal amplification, and microarray analysis, as well as immunological analysis processes and bacterial growth analysis. Further, chemical reactions include processes that analyze the generation, transformation, or destruction of chemicals. According to an embodiment, the signal generation reaction may be a genetic analysis process, or may be a nucleic acid amplification reaction, an enzyme reaction, or microbial growth.

Meanwhile, the aforementioned signal generation reaction accompanies a change in the signal. Therefore, the degree of progress of such a signal generation reaction may be evaluated by measuring the change in the signal.

Here, the term "signal" refers to a measurable output. Further, the measured magnitude or change of such a signal serves as an indicator that qualitatively or quantitatively indicates the characteristics of the target analyzed substance, specifically the presence or absence of the target analyzed substance in the sample.

Here, examples of indicators include fluorescence intensity, luminescence intensity, chemiluminescence intensity, bioluminescence intensity, phosphorescence intensity, charge movement, voltage, current, power, energy, temperature, viscosity, light scatter, radioactivity, reflectance, transmittance, and absorbance, but are not limited thereto.

The term "signal generation means," as described above, refers to the means that provide a signal indicating the properties of the target analyzed substance to be analyzed, specifically the presence or absence of the analyte.

The signal generation means include the label itself or an oligonucleotide to which the label is attached.

Among these, the labels include fluorescent labels, luminescent labels, chemiluminescent labels, electrochemical labels, and metal labels. The label may be used as the label itself, such as an intercalating dye. Alternatively, the label may be in the form of a single label or an interactive dual label including a donor molecule and an acceptor molecule, and used in the form of being coupled to one or more oligonucleotides.

In case of using a fluorescent label, the signal value may be expressed as a relative fluorescence unit (RFU) value.

The signal generation means may further include an enzyme with nucleic acid cleavage activity to generate the signal (e.g., an enzyme with 5' nucleic acid cleavage activity or an enzyme with 3' nucleic acid cleavage activity).

Meanwhile, various methods are known for organizing signals that indicate the presence of the target analyzed substance, particularly target nucleic acid molecules, using the signal generation means. Representative examples may include the following: TaqMan^{™} probe method (U.S. Patent No. 5,210,015), molecular beacon method (Tyagi, Nature Biotechnology, v.14, March 1996), Scorpion method (Whitcombe et al., Nature Biotechnology 17:804-807, 1999), Sunrise (or Amplifluor) method (Nazarenko et al., Nucleic Acids Research, 25(12):2516-2521, 1997; U.S. Patent No. 6,117,635), Lux method (U.S. Patent No. 7,537,886), CPT (Duck P., et al., Biotechniques, 9:142-148, 1990), LNA method (U.S. Patent No. 6,977,295), Plexor method (Sherrill CB, et al., Journal of the American Chemical Society, 126:4550-4556, 2004), Hybeacons (D.J. French, et al., Molecular and Cellular Probes 13:363-374, 2001; U.S. Patent No. 7,348,141), dual-labeled self-quenched probe (U.S. Patent No. 5,876,930), hybridization probe (Bernard PS, et al., Clin Chem 2000, 46, 147-148), PTOCE (PTO cleavage and extension) method (WO 2012/096523), PCE-SH (PTO Cleavage and Extension-Dependent Signaling Oligonucleotide Hybridization) method (WO 2013/115442), PCE-NH (PTO Cleavage and Extension-Dependent Non-Hybridization) method (PCT/KR2013/012312), and CER method (WO 2011/037306).

Meanwhile, the aforementioned term "signal generation reaction" may include the amplification reaction of the signal. In this case, the term "amplification reaction" refers to a reaction that increases or decreases the signal generated by the signal generation means. Specifically, the amplification reaction refers to a signal increase (or amplification) reaction generated by the signal generation means, which is dependent on the presence of the target analyzed substance.

In such an amplification reaction, the amplification of the target analyzed substance (e.g., nucleic acid molecule) may or may not be accompanied. More specifically, the amplification reaction may refer to a signal amplification reaction that accompanies the amplification of the target analyzed substance.

Meanwhile, the data set obtained through the amplification reaction may include amplification cycles.

Here, in at least two measurements involving changes in specific conditions, the term "cycle" refers to the unit of change in the conditions. The change in the specific conditions refers to, for example, an increase or decrease in temperature, reaction time, number of reactions, concentration, pH, or the number of replications of the measurement target (e.g., nucleic acids). Therefore, a cycle may refer to a time or process cycle, a unit operation cycle, or a reproductive cycle.

More specifically, the term "cycle" refers to one unit of repetition in cases where a reaction of a specific process is repeated or a reaction is repeated based on a specific time interval.

Alternatively, the term "cycle" may refer to one unit of repetition in cases where a specific action is repeated according to the progression of the reaction.

As an example, when a nucleic acid amplification reaction is performed, the action of detecting the signal occurring at specific time intervals may be repeated, and this repetition may refer to one unit of the cycle. In this case, the cycle may have a unit of time.

As an example, in case of a nucleic acid amplification reaction, one cycle refers to a reaction that includes the denaturation step, primer annealing step, and primer extension step. In this case, the change in specific conditions refers to an increase in the number of reaction repetitions, and the repetition unit of the reaction, which includes a series of steps described above, is set as one cycle. The number of cycles may include the number of reaction repetitions or the reaction time.

Meanwhile, the target analyzed substance or target analyte, particularly the target nucleic acid molecule, may be amplified using various methods: polymerase chain reaction (PCR), ligase chain reaction (LCR) (U.S. Patents No. 4,683,195 and 4,683,202; PCR Protocols: A Guide to Methods and Applications (Innis et al., eds, 1990)), strand displacement amplification (SDA) (Walker et al., Nucleic Acids Res. 20(7):1691-6, 1992; Walker, PCR Methods Appl 3(1):1-6, 1993), transcription-mediated amplification (Phyffer et al., J. Clin. Microbiol. 34:834-841, 1996; Vuorinen et al., J. Clin. Microbiol. 33:1856-1859, 1995), nucleic acid sequence-based amplification (NASBA) (Compton, Nature 350(6313):91-2, 1991), rolling circle amplification (RCA) (Lisby, Mol. Biotechnol. 12(1):75-99, 1999; Hatchett et al., Genet. Anal. 15(2):35-40, 1999), Q-beta replicase (Q-Beta Replicase) (Lizardi et al., Biotechnology 6:1197, 1988), loop-mediated isothermal amplification (LAMP, Y. Mori, H. Kanda and T. Notomi, J. Infect. Chemother., 2013, 19, 404-411), recombinase polymerase amplification (RPA, J. Li, J. Macdonald and F. von Stetten, Analyst, 2018, 144, 31-67).

Meanwhile, the amplification reaction amplifies the signal as it accompanies the amplification of the target analyzed substance (specifically, the target nucleic acid molecule). For example, the amplification reaction may be performed according to PCR, specifically real-time PCR, or may be conducted as an isothermal amplification reaction (e.g., LAMP or RPA).

Meanwhile, the data set obtained by the signal generation reaction includes at least two data points, which include the cycles of the signal generation reaction and the signal values at these cycles.

Here, the term "signal value" refers to the value or its variation value obtained by quantifying the level of the signal (e.g., signal intensity) actually measured during the cycles of the signal generation reaction, particularly the amplification reaction, according to a predetermined scale. The variation value may include the mathematically processed signal value derived from the actual measured signal value. Examples of mathematically processed signal values derived from the actual measured signal values (i.e., the signal values of the raw data set) include logarithmic values or derivatives.

The term "data point" refers to a single coordinate value that includes both the cycle and the signal value. Further, the term "data" refers to all the information that constitutes the data set. For example, the cycles of the amplification reaction and the signal values may each correspond to data.

The data points obtained from the signal generation reaction, particularly the amplification reaction, may be displayed as coordinate values that can be represented in a two-dimensional Cartesian coordinate system. In the coordinate values, the X-axis represents the number of cycles, and the Y-axis represents the signal value measured or processed in the corresponding cycle.

The term "data set" refers to a collection of data points. For example, a data set may be a collection of data points directly obtained through an amplification reaction performed in the presence of the signal generation means, or may be a modified data set in which such a data set has been modified. The data set may be some or all of the at least two data points obtained through the amplification reaction or their modified data points.

Meanwhile, the data set may also be a data set obtained by processing at least two data sets. In case of performing analysis on at least two target analyzed substances in a single reaction vessel, in some cases, the data set for the at least two target analyzed substances may be obtained through the processing of the data sets obtained from the reaction performed in the single reaction vessel. For example, the data set for at least two target analyzed substances performed in a single reaction vessel may be obtained by processing at least two data sets obtained from signals measured at different temperatures.

The aforementioned data set may be plotted, and thereby an amplification curve may be obtained.

The term "plate" refers to a basic unit in an amplification device where the amplification reaction is performed, and it also refers to a basic unit where the data generated after the amplification reaction is stored. Different plates may be plates where amplification reactions are performed at different times using the same amplification device, or plates where amplification reactions are performed at the same time by different amplification devices.

The plate includes at least two reaction wells. The plate may include N x M reaction wells. Typically, the plate includes 12 x 8 or 8 x 12 reaction wells. The reaction wells of the plate may be either integrated with the plate or in the form of detachable tubes. The plate may be rectangular in shape, but the plate may also include one or more reaction wells and may be implemented in various forms other than rectangular, such as circular, trapezoidal, rhombic, or other shapes.

The wells of the plate include the samples to be analyzed and the reagents necessary for the nucleic acid amplification reaction.

With reference to the following drawings, various embodiments of the present invention will be described.

FIG. 1 illustrates a computer device 100 according to an embodiment, along with a nucleic acid detection device 200 connected thereto and database 300. These 100, 200, and 300 may be connected to each other via wired or wireless communication. However, FIG. 1 is merely illustrative, and the ideas of the present disclosure are not limited to those illustrated in FIG. 1. For example, the components that are not illustrated in FIG. 1 may be additionally connected to these 100, 200, and 300. These may include a server that derives candidate nucleotides in-silico, a preparation device that performs the preparation process for nucleic acid amplification, or a result analysis module that reads whether a specific target analyte is positive or negative based on the results detected by the nucleic acid detection device 200, or visualizes and provides the detected results as a specific graph. However, this is not limited to these components.

Meanwhile, unlike what is illustrated, the database 200 may be implemented by being included in the computer device 100. However, the following description will be based on the assumption that the aforementioned components 100, 200, and 300 are implemented or connected as illustrated in FIG. 1. Hereinafter, each component will be examined in detail.

The nucleic acid detection device 200 is implemented to perform the nucleic acid amplification reaction and nucleic acid detection tasks on the sample. According to the embodiment, the nucleic acid detection device 200 may be implemented to perform the nucleic acid detection task without performing the nucleic acid amplification reaction. However, the following description will be explained based on the assumption that the nucleic acid detection device 200 is implemented to also perform the nucleic acid amplification reaction.

Meanwhile, as the aforementioned nucleic acid amplification reaction is performed, if the sample in the reaction vessel includes the target nucleic acid molecule, its quantity will be amplified, and as previously mentioned, the magnitude of the signal generated by the signal generation means may also be amplified. Thus, the nucleic acid detection device 200 is implemented to detect the magnitude of such a signal. Specifically, the nucleic acid detection device 200 may real-time monitor the magnitude of the signal that changes as the nucleic acid amplification reaction progresses. The monitored results may be output from the nucleic acid detection device 200 in the form of a data set, as mentioned in the definition of the term, for example, as the signal intensity for each cycle. Here, the signal intensity for each cycle is the information that is the basis for reading the presence or absence of the target nucleic acid molecule in the corresponding sample.

Meanwhile, since the magnitude of the signal generated by the signal generation means may also be amplified when the nucleic acid amplification reaction is performed, the nucleic acid amplification reaction is considered below to cause the signal generation reaction that was previously examined as a term.

The database 300 stores various types of data.

For example, the database 300 may store data on the aforementioned nucleic acid amplification reaction. Specifically, the signal intensity for each of the at least two cycles for amplifying the nucleic acid, the form of the amplification curve generated using the signal intensity for each cycle, the positive/negative read results, or the like may be stored as research and development data. In this specification, the term "data on nucleic acid amplification reaction" may include result data of an amplification reaction.

In addition, meta-data for such research and development data may also be stored in the database 300. Meta-data may be described as "data on data," that is, data that provides information on other data. For example, at least one of information on the oligonucleotide itself, the types of performance verification experiments to be performed on this oligonucleotide, the materials used in the experiment, or the experimental procedures and experimental devices used in the experiment may be included in the meta-data. Here, the aforementioned items that may be included in the meta-data will be examined in more detail below.

In addition, in this disclosure, the oligonucleotide may include at least two candidate oligonucleotides, and may include the oligonucleotide selected among these at least two candidate oligonucleotides to finally be included in the detection reagent for a target nucleic acid molecule.

The computer device 100 provides an interface for the input of the aforementioned research and development data or the aforementioned meta-data. By providing such an interface, first, the data (hereinafter, "data" collectively refers to both research and development data and meta-data) may be stored without omission. There are many types of data that need to be managed for research and development, and depending on the situation, there is a risk that some data may not be input and be omitted. However, in an embodiment, by providing such an interface through the research and development management device 100, the researcher or developer may input data that requires input on the interface. This ensures that the possibility of data input omission is eliminated or reduced.

Second, the aforementioned data may be stored in a structured format, that is, normalized. These data can be reused to improve or modify the corresponding research and development, or for another research and development. In this case, for smooth reuse, these data need to be stored according to a predefined standard. In this regard, in an embodiment, during the input process of these data or the process of storing these data, the data may be stored in a structured format, that is, in a standardized state.

Third, the aforementioned data may be easily searched. Specifically, in an embodiment, the search for the aforementioned data may be performed according to the conditions desired by the researcher or developer. For example, these conditions may include the type or concentration of the candidate nucleotides, the type and concentration of the target analyzed substance, the type of consumables, the manufacturer information for the enzyme and buffer, the duration of the experiment, the person who performed the experiment, or the like, but these are not limited thereto.

Hereinafter, the device 100 according to the present disclosure will be described in more detail.

FIG. 2 is a block diagram of the computer device 100 according to an embodiment.

With reference to FIG. 2, the computer device 100 includes a communication unit 110, a memory 120, a processor 130, and a display unit 140, but is not limited thereto.

First, the communication unit 110 is implemented as a wired or wireless communication module. Through the communication unit 110, the computer device 100 may perform communication with external sources. For example, the computer device 100 may receive result data of an amplification reaction from the nucleic acid detection device 200 from the communication unit 110. Additionally, the computer device 100 may receive information necessary for research and development data management from external sources, for example, from the database 300 illustrated in FIG. 1.

The memory 120 stores various types of data or information, including at least one command. The stored data may include data received through the communication unit 110 from the database 300 or data processed by the processor 130. These data or information may include various types of research and development information, etc. necessary for research and development data management.

Meanwhile, in FIG. 2, the memory 120 is illustrated as a separate component from the processor 130, but the memory 120 may also be implemented as a single device with the processor 130. For example, the memory 120 may be a storage, such as a cache, included within the processor 130.

Next, the processor 130 may be implemented by a central processing unit (CPU), graphics processing unit (GPU), micro controller unit (MCU), a dedicated processor on which the methods according to an embodiment are performed, or the like. Hereinafter, the processor 130 may collectively refer to a single processor or at least two processors, for example, a multi-core processor.

The processor 130 may write data to the memory 120. Additionally, the processor 130 may fetch and execute instructions stored in the memory 120. For example, by executing the instructions stored in the memory 120, the processor 130 enables the research and development data management device 100 to perform the functions described below, which will be explained subsequently.

The display unit 140 may be driven by the processor 130 as follows.

First, the display unit 140 may display information. In addition, through the display unit 140, the user may input specific information. For such display or input, the display unit 140 may be implemented as a touchscreen or touchpad, or alternatively, may be implemented through a combination of an LCD monitor, a keyboard, and others. Here, the information displayed on the display unit 140 may be something that the research and development data management device 100 has received from an external source via the communication unit 110 or may be loaded from the memory 120, but is not limited thereto.

According to the embodiment, the display unit 140 displays an input screen for receiving research and development data or meta-data. The details or content included in such a screen may be something that the computer device 100 has received from an external source, such as the database 300, through the communication unit 110, or may be loaded from the memory 120, but is not limited thereto.

The types of research and development data or meta-data input into the display unit 140 have been described earlier, and these will be referenced here.

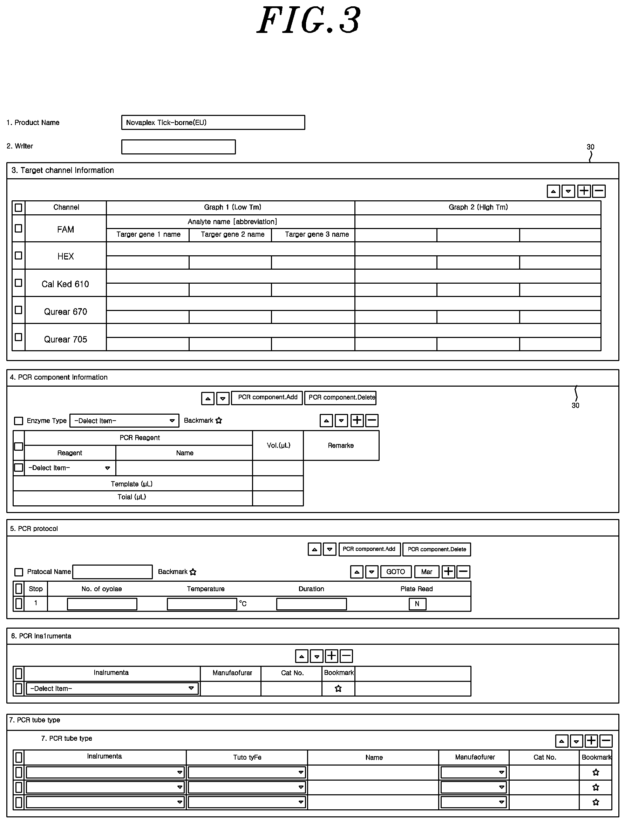

Hereinafter, the respective functional configurations of the computer device 100 that provides such a data input screen will be described with reference to FIG. 3 to FIG. 9 in more details.

The research computer device 100 according to an embodiment may guide at least two detailed performance experiments required for the development of a detection reagent for a target nucleic acid molecule, and may provide the organization of an experimental result documentation for each detailed performance experiment according to the at least two detailed performance experiments.

To this end, the computer device 100 according to the present disclosure may provide a shared data input screen for at least one requirement item for performing an experiment selected among the requirement items for performing an experiment (item for experimentation), including materials, experimental procedures, experimental devices, and a combination thereof used in at least two detailed performance experiments, as illustrated in FIG. 3.

Via the shared data input screen illustrated in FIG. 3, detailed data corresponding to requirement items for performing an experiment, including materials, experimental procedures, experimental devices, and a combination thereof commonly used in at least two detailed performance experiments for the development of a detection reagent for a target nucleic acid molecule, may be input.

The shared data input via this shared data input screen may be provided as input data to the input data screen of requirement items for performing an experiment (item for experimentation) for each detailed performance experiment, based on the user's selection, through a user input, as described below.

Oligonucleotide is used to detect a target nucleic acid in a target nucleic acid molecule by polymerase chain reaction (PCR), and refers to a subject of a test for whether detection performance of the target nucleic acid molecule satisfies a predetermined level or for evaluating/optimizing the detection performance.

FIG. 3 is a shared data input screen provided on the display unit 140 of the computer device 100 according to an embodiment. With reference to FIG. 3, a shared data input screen for the input of basic information on the candidate oligonucleotide and/or shared data for research and development on the detection reagent for a target nucleic acid molecule to be developed is exemplarily illustrated. In the shared data input screen illustrated in FIG. 3, at least one experimental materials-related data selected among the group consisting of the type and concentration of the oligonucleotide, the type and concentration of the target nucleic acid molecule, the type of enzyme, buffer, type of reaction vessel, type and concentration of positive control, type and concentration of negative control, type and concentration of internal control, type and amount in use of water, and a combination thereof may be input.

In the shared data input screen, at least one experimental procedures-related data input in the shared data input screen is selected among the group consisting of information on the target nucleic acid molecule for each detection channel (FAM, HEX, Cal Red 610, Quasar 670, Quasar 705), amplification reaction protocol, extraction method of the target nucleic acid molecule, and a combination thereof may be input.

Specifically, information regarding the selected detection reagent for a target nucleic acid molecule, the author, the configuration of the panel where the amplification reaction is performed in the nucleic acid detection device 200 by accommodating each of at least two candidate oligonucleotides for each well, PCR components, PCR protocol, PCR equipment, and PCR consumables may be input.

For example, in a target channel information table 30, the target gene names for each channel at either high or low temperature may be input for the target analyte, and in a PCR component information table 31, information on the type or volume of the enzyme, buffer, and template may be input.

Additionally, at least one experimental procedures -related data input in the shared data input screen is selected among the group consisting of nucleic acid detection devices, experimental preparation devices, and a combination thereof may be input. The data input via this shared input screen is stored in a structured preset format. For example, data input in a non-structured format may be converted into a structured format, or the input itself may be rejected. Specifically, when inputting PCR consumables, in case that a typo occurs or a consumable not included in the predefined list is input, the input thereof may be rejected. Alternatively, in case of the PCR protocol, the number of cycles and the temperatures at which they are performed need to be designated. A format for designating these cycles and temperatures exists, and the PCR protocol input in accordance with this format may be stored in a structured format, that is, in a normalized manner.

The data input via the shared input screen according to the present disclosure is the data collected in the step of providing the shared input screen, which may be converted into predefined results and stored as the experiment result documentationexperiment result documentation described below.

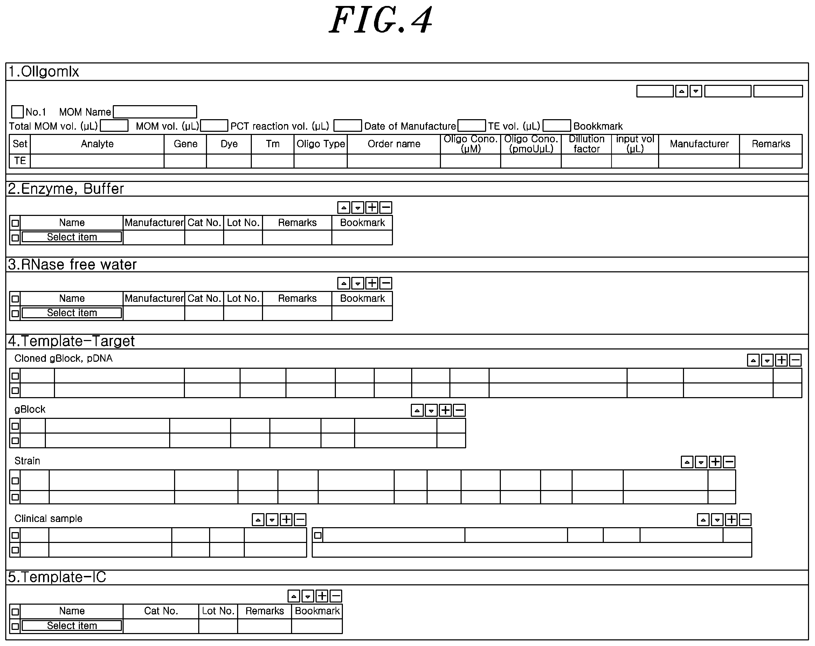

FIG. 4 is another input screen provided on the display unit 140 of the computer device 100 according to an embodiment. With reference to FIG. 4, a shared data input screen for inputting material information used in at least two detailed performance experiments performed on the oligonucleotide for the research and development of the detection reagent for a target nucleic acid molecule is exemplarily illustrated.

The materials used in the experiment include at least one requirement item for performing an experiment selected among the requirement items for performing an experiment including clinical samples, types of clinical samples, types of labels, the type and concentration of the candidate oligonucleotide to be used in each experiment, the type and concentration of the target nucleic acid molecule, types of consumables, manufacturer information for the enzyme and buffer, and a combination thereof.

For example, an embodiment of a shared data input screen for inputting material information on Mastermix including oligonucleotides and the template diluted in the Mastermix, and information on the extraction method is illustrated.

The shared data input screens shown in FIGS. 3 and 4 may also be displayed separately as the info. and Mtrls. tabs, respectively, depending on the embodiment.

Next, Oligomix and Mastermix, which will be described below, will be examined.

Oligomix refers to oligonucleotides in which a forward primer, a reverse primer, and a probe are mixed.

Mastermix refers to a solution in which enzyme, TE buffer, and RNase-free water are mixed with the Oligomix.

Specifically, in the shared data input screen of FIG. 4, material information may be input regarding the total volume of the Oligomix, the volume occupied by the Oligomix in the Mastermix in which the Oligomix, TE buffer, and enzyme are mixed, the concentration values of oligonucleotides by type, the volume of Oligomix dispensed into at least one vessel, the dilution factor that determines a setting concentration based on the basic concentration of oligonucleotide by type, the names of oligonucleotides by type, the oligonucleotide type for each oligonucleotide name, and the like. In this case, the Oligomix refers to oligonucleotides in which a forward primer, a reverse primer, and a probe are mixed.

In addition, in the shared data input screen of FIG. 4, information on the manufacturer, Cat No., Lot No., etc. of reagents such as enzymes, buffers, and RNase-free water mixed with oligonucleotides during the manufacture of Mastermix may be input.

Further, information on the concentration, Cat No., Lot No., etc. for each template type, information regarding the positive control (PC), and the extraction method may be input.

As described above, in the data input screens illustrated in FIG. 3 and FIG. 4, basic information on the oligonucleotide and material information on the oligonucleotide and the reagents mixed with the oligonucleotide may be input for research and development of the detection reagent for a target nucleic acid molecule, according to an embodiment.

However, the illustrated screens are merely embodiments according to the present disclosure and are not limited thereto.

In this disclosure, the oligonucleotide refers to a subject of a test for whether the detection performance satisfies a predetermined level or for optimizing the detection performance, and the test guides at least two detailed performance experiments with different performance to be validated.

Since the performance of the oligonucleotide may vary under different conditions, tests for each material constituting the oligonucleotide need to be performed very strictly. Therefore, in case of these oligonucleotides, a test of their performance needs to be performed to determine whether they are able to function well under various conditions.

The computer device 100 according to the present disclosure may request data on requirement items for performing an experiment, including materials, experimental procedures, experimental devices, and a combination thereof used in the development of the detection reagent for a target nucleic acid molecule, via the shared data input screen illustrated in FIG. 3 and FIG. 4, in order to guide each of the at least two detailed performance experiments for the user who intends to develop the corresponding detection reagent for a target nucleic acid molecule.

Each of the at least two detailed performance experiments is a test for the development of a specific detection reagent for a target nucleic acid molecule, but they are distinct from one another, and the material information required for each detailed performance experiment may also differ. However, since each of these detailed performance experiments is a test for a specific detection reagent for a target nucleic acid molecule, the basic information is commonly based on the specific detection reagent for a target nucleic acid molecule.

The described shared data input screen illustrated in FIG. 3 and FIG. 4 is an example of a common data input screen for receiving shared data, which is commonly applied identically despite different detailed performance experiments on the oligonucleotide.

Therefore, the basic information and material information input via the shared data input screen illustrated in FIG. 3 and FIG. 4 may be the information that is commonly and identically applied to detailed performance experiments with different performance to be validated in the at least two detailed performance experiments to be performed on oligonucleotides.

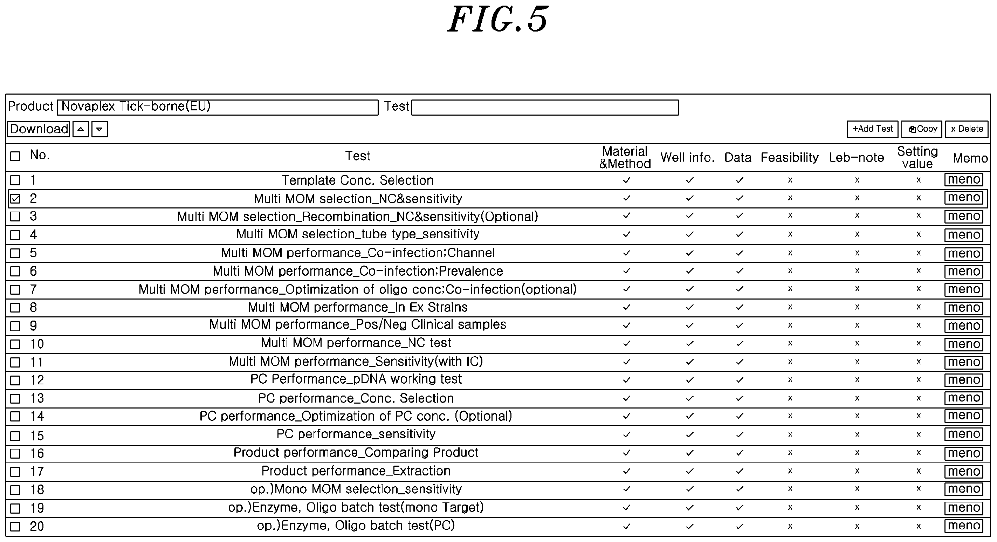

The at least two detailed performance experiments according to the present disclosure will be examined in detail with reference to FIG. 5.

FIG. 5 exemplarily illustrates the screen provided by the computer device 100 to allow for the selection which performance experiment to perform among the at least two detailed performance experiments on the oligonucleotide according to an embodiment.

The computer device 100 according to an embodiment may display a detailed performance experiment input screen, as illustrated in FIG. 5, allowing for the selection of the type of detailed performance experiment.

Therefore, the computer device 100 according to the present disclosure may distinguish between common information based on all the at least two detailed performance experiments for the detection reagent for a target nucleic acid molecule, which is input via the shared data input screen, and unique information for each detailed performance experiment, which is input via the detailed performance experiment input screen.

In this case, each detailed performance experiment is for testing the performance of the oligonucleotide, and the performance of the oligonucleotide may include sensitivity and/or specificity for the target nucleic acid molecule.

For such detailed performance experiments, the computer device 100 according to an embodiment may provide a data input screen for at least one requirement item for performing an experiment, selected among the requirements item for performing an experiment (item for experimentation) including materials, experimental procedures, experimental devices, and a combination thereof used in the detailed performance experiments performed on the oligonucleotide for detecting the target nucleic acid molecule.

As illustrated in FIG. 5, the types of at least two detailed performance experiments according to the present disclosure may include at least one group selected among the group consisting of target nucleic acid molecule concentration selection, oligonucleotide selection_NC and sensitivity, recombination, oligonucleotide performance sensitivity, oligonucleotide performance optimization, PC performance sensitivity, and a combination thereof.

Meanwhile, in FIG. 5, the computer device 100 allows for checking and confirming the progress of the detailed procedures for each detailed performance experiment.

The detailed procedures for each detailed performance experiment may include, as illustrated in FIG. 5, the following items: Material & Method, well info, Data, DSP, Lab-note, and Setting value. Accordingly, the computer device 100 according to the present disclosure may allow for the selection of the types of at least two detailed performance experiments via the detailed performance experiment input screen and/or for confirming the progress of the detailed procedures for each detailed performance experiment.

The detailed procedures according to an embodiment may include, as illustrated in FIG. 5, requirement items for performing an experiment (Material & Method), preparation information (Well info), result data reception of the amplification reaction (Data), processed/analyzed data obtaining (DSP), and experimental result documentation(e.g., Lab-note).

The progress status for each of these requirement items for performing an experiment, preparation information, result data reception of an amplification reaction, processed/analyzed data obtaining, and experimental result documentation may be provided via sections on the detailed performance experiment input screen, where they are displayed with icons (√, X), respectively.

When an icon is selected, a screen may be provided that displays the detailed contents of the corresponding requirement items for performing an experiment, preparation information, result data of an amplification reaction, processed/analyzed data, or experimental result documentation.

According to an embodiment, the detailed procedures may include at least one requirement item for performing an experiment selected among the requirement items for performing an experiment including materials used in the experiment, experimental planning, experiment preparation, experimental devices, experimental procedures, and a combination thereof, as well as the organization of the experimental results. In the screen illustrated in FIG. 5, this may be displayed as Material & Method, Well info, Data, Feasibility, Lab-note, and Setting value.

However, the detailed performance experiment input screen according to the present disclosure may undergo various modifications and alterations without departing from the intrinsic characteristics of the disclosure. Therefore, the embodiments disclosed in the present disclosure are intended to explain, not to limit, the technical scope of the present disclosure, and the technical scope of the present disclosure is not limited by the embodiments. The computer device 100 according to the present disclosure, as illustrated in the detailed procedures for each detailed performance experiment in FIG. 5, once the requirement items for performing an experiment (Material & Method) for any one detailed performance experiment selected among at least two detailed performance experiments are created, will display the preparation information for the reaction plate on the display of the computer device 100 based on this creation. Based on the displayed preparation information and requirement items for performing an experiment, the computer device 100 may command the linked experimental preparation device to drive in order to manufacture the amplification composition including the oligonucleotide. As will be described below, in this process, the computer device 100 may display a parameter input screen on the display, where the values of the parameters necessary for driving the experimental preparation device may be input. When the amplification composition including the oligonucleotide is manufactured based on the values of the parameters input via the parameter input screen, the amplification reaction of the reaction plate for this is performed through the nucleic acid detection device 200, and the result data of amplification reaction as a result of the performance is uploaded to the computer device 100. This may be confirmed in the Data item of the detailed procedures. When the result data of amplification reaction for a specific detailed performance experiment exported by the nucleic acid detection device 200 is selected in the Data item, the processing/analysis of the selected result data of amplification reaction may be performed through the selection of the DSP item in the detailed procedures. In the DSP item, the processing/analysis is performed to obtain processed/analyzed data for the result data of amplification reaction corresponding to a specific detailed performance experiment. The processing/analysis is performed for each detailed performance experiment, and by setting the setting values, which include the necessary values for processing/analysis (e.g., reference value and/or signal value-related parameters (Cut-off ratio, CR)) into the corresponding result data of amplification reaction, the processed/analyzed data may be obtained. The results input and/or performed in these items according to the detailed procedures are all converted into a preset format and may be stored in the lab-note item in the experimental result documentation for each detailed performance experiment.

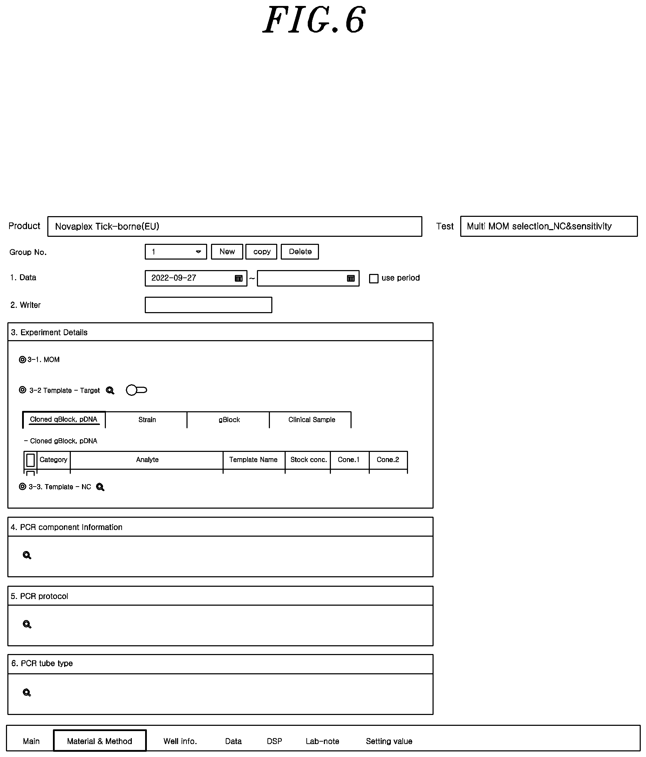

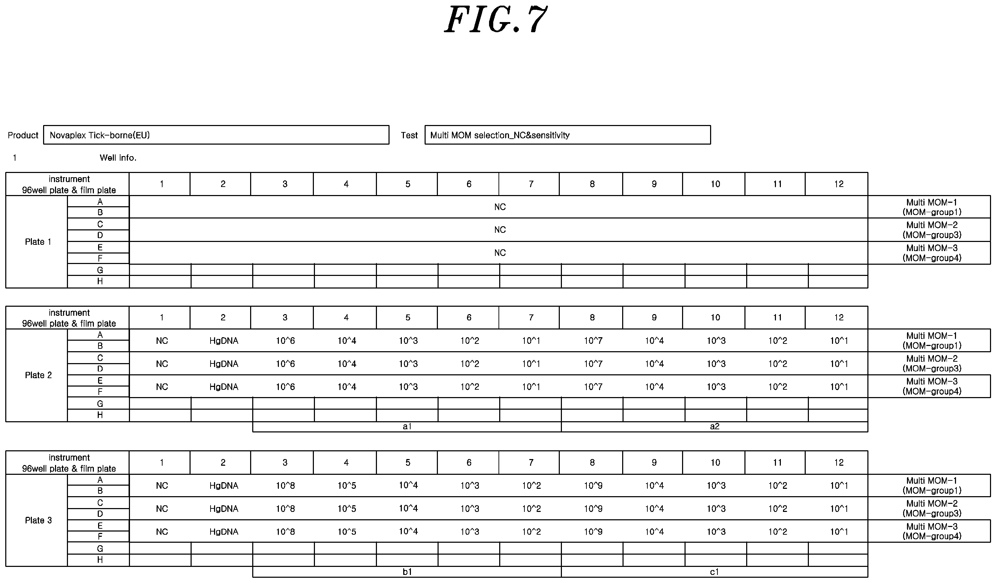

FIG. 6 and FIG. 7 exemplarily illustrate an embodiment of screens provided for inputting requirement items for performing an experiment for each detailed performance experiment according to an embodiment.

First, in FIG. 6, each of the tabs at the bottom of the drawing displays the detailed experimental procedures described above, and they are intended to indicate which specific detailed experimental procedure screen from those illustrated in FIG. 5 is being referred to. For example, the activated Material & Method item in the tabs below corresponds to the requirement items for performing an experiment, unlike the common data input screen for shared data input illustrated in FIG. 3 and FIG. 4, is a data input screen for the requirement items for performing an experiment, provided for each detailed performance experiment, in order to provide a unique data input screen for each detailed performance experiment.

Specifically, as an item for inputting oligonucleotide and material information for the oligonucleotide in the corresponding detailed performance validation experiment, at least one requirement item for performing an experiment selected among the requirement items for performing an experiment that includes the type and concentration of the oligonucleotide, the type and concentration of the target nucleic acid molecule, types of consumables, manufacturer information for the enzyme and buffer, and a combination thereof, for each detailed performance experiment, may be input.

The requirement items for performing an experiment according to an embodiment are based on the basic information and material information for the oligonucleotide input via the shared data input screens in FIG. 3 and FIG. 4, but are intended to input information that is specifically further added for the corresponding detailed performance experiment. The at least two detailed performance experiments are distinct from each other and are attributable to the specific characteristics of each experiment. For example, the requirement items for performing an experiment (Material & Method) are intended to input additional information for each detailed performance experiment or unique information in the corresponding detailed performance experiment, due to the cases where existing materials are discarded and new materials are used, where the Lot is changed, or the like, each time when each of the at least two detailed performance experiment is proceeded.

In these requirement items for performing an experiment, when the information input via the shared data input screens in FIG. 3 and FIG. 4 overlaps with the content being input for the corresponding detailed performance experiment, the information input via the shared data input screens in FIG. 3 and FIG. 4 may also be retrieved and input via a separate icon ( 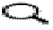 ) to avoid redundant input.

The computer device 100 according to the present disclosure displays preparation information for the reaction plate, which is used by the nucleic acid detection device 200 to perform the amplification reaction using the oligonucleotide, based on the data input via the data input screen for the requirement items for performing an experiment for each detailed performance experiment.

According to an embodiment, the preparation information display refers to displaying at least one of the type of template, and/or the concentration or series of concentrations of the template, the type of oligonucleotide, and the type of reaction plate, which is suitable for the purpose of the detailed performance experiment.

Each of the at least two reaction vessels included in the reaction plate may display the type of template and/or the concentration or series of concentrations of the template.

The preparation information (Well info) illustrated in FIG. 7 is intended to display information on the reaction plate, which includes a reaction vessel prepared based on the information input in the requirement items for performing an experiment, for example, at least two reaction vessels (e.g., wells). That is, the preparation information is generated and displayed based on the information input in the requirement items for performing an experiment item for the experimental planning in the detailed procedures.

In this case, the reaction plate is a reaction plate on which the amplification reaction is actually performed through the nucleic acid detection device 200 with the corresponding detailed performance experiment prepared.

As described, the preparation information is intended for displaying information on the reaction plate prepared for each of the at least two detailed performance experiments. The information displayed for each reaction vessel of the reaction plate is dispense information for each well used when performing the test for each detailed performance experiment, based on the data input information for the requirement items for performing an experiment for each corresponding detailed performance experiment.

In the computer device 100 according to the present disclosure, in order to display the reaction plate image in which the dispense information for the corresponding preparation information for each detailed performance experiment is displayed, the type and concentration values of the target nucleic acid molecule may be displayed for each well based on the information input for the requirement items for performing an experiment for each detailed performance experiment, on the default plate image preset for each detailed performance experiment.

In this case, the preset default plate image is changed dependently according to the factors preset for each of the at least two detailed performance experiments. The preset factors may include at least one of the types of oligonucleotides, the template, the concentration of the template, and the types of plates.

The researcher or developer using the computer device 100 according to the present disclosure may prepare the reaction plate according to the preparation information prepared for each of the at least two detailed performance experiments, that is, the reaction plate image displayed in the preparation information, based on the information input via the data input screen for the requirement items for performing an experiment.

Meanwhile, in order to optimize the performance of the oligonucleotide to be included in the detection reagent for a target nucleic acid molecule, it is desirable to manufacture various candidate oligonucleotides for detection by changing the conditions and environments of each material, and based on this, select a suitable amplification composition for detection from the manufactured candidate amplification compositions for detection.

Accordingly, in the present disclosure, at least one candidate amplification composition for detection may be manufactured to select the oligonucleotide to be included in the detection reagent for a target nucleic acid molecule, or the experimental preparation device may be linked to manufacture an amplification composition for detection that includes the oligonucleotide to be included in the detection reagent for a target nucleic acid molecule from the candidate amplification compositions for detection.

To this end, the computer device 100 may provide a parameter input screen for the values of parameters for detailed tasks for each manufacturing task required for driving the experimental preparation device that manufactures the amplification composition needed for the detailed performance experiment.

The manufacturing task may include at least one of the detailed task of template dilution used in the amplification reaction, the detailed task of the oligonucleotide mixture, the detailed task of the Master mixture including the enzyme, buffer, and the manufactured oligonucleotide mixture, or the detailed task of setting up the amplification reaction solution by dispensing the manufactured Master mixture and the template dilution into the reaction vessel.

The aforementioned detailed tasks may be selected via the input screen required for the manufacture of the candidate amplification composition/amplification composition for detection. To perform the detailed task selected via such an input screen, the values of the parameters required for the detailed task may be input via the parameter input screen.

In this case, the values of the parameters input into the parameter input screen may be automatically input in linkage with the data input via the data input screen for the requirement items for performing an experiment.

The values of the parameters may have various characteristics. For example, the values of the parameters may include information on the type, concentration, and volume of the material used in the detailed task of the candidate amplification composition for detection, the position where the vessel accommodating the material is disposed on the deck of the experimental preparation device, and the position where the reaction vessel accommodating the candidate amplification composition for detection is disposed on the reaction plate of the experimental preparation device.

Therefore, the values of the parameters input into the parameter input screen may include the preparation information according to the present disclosure. Based on the preparation information, for example, candidate amplification compositions for detection may be manufactured, and the reaction plate for the amplification reaction may be prepared, based on the type of template and/or the concentration or series of concentrations of the template.

The reaction plate for the candidate oligonucleotide amplification composition for detection, for which each detailed task has been completed, may perform the amplification reaction in the nucleic acid detection device 200.

Accordingly, the computer device 100 according to an embodiment may receive the result data of amplification reaction performed by the nucleic acid detection device 200 using the amplification composition including the oligonucleotide prepared according to the preparation information for each detailed performance experiment based on the requirement items for performing an experiment and/or the preparation information.

The amplification result data for this may be accessed through the Data item described below.

In the detailed procedures for each of the at least two detailed performance experiments, the Data item is intended to display information on the data exported from the nucleic acid detection device 200 for each of the at least two detailed performance experiments. In this case, the data may be a raw data set. The raw data set refers to a collection of data points that include the cycle number and signal values obtained from the signal-generation reaction. The raw data set refers to a group of unprocessed data points initially obtained from an equipment (e.g., a nucleic acid detection device), for performing a signal-generation reaction like real-time PCR.

According to the embodiment, the data exported from the nucleic acid detection device 200 for each of the at least two detailed performance experiments may be a data set with the baseline subtracted from the raw data set in order to remove the background signal values. The baseline-subtracted data set may be obtained through various methods known in the art (e.g., U.S. Patent No. 8,560,247).

In the Data item, information on the name of the nucleic acid detection device that performed the detailed performance experiment, the file upload status of the raw data set, tube type, and others may be input.

According to the embodiment, the Data item may provide information on the device name of the nucleic acid detection device 200 received by the computer device 100, the receipt status of the result data of amplification reaction, and the type of consumables used in the corresponding device.

Next, the computer device 100 according to an embodiment may process/analyze the result data of amplification reaction received from the nucleic acid detection device 200 to obtain the processed/analyzed data.

To this end, in the detailed procedures for each of the at least two detailed performance experiments, the DSP item provides an interface used to set the value of parameters used in the process of reading the presence or absence of the target nucleic acid molecule.

According to an embodiment, in the DSP item, the analysis of the amplification reaction result values and the setting of setting values for the result data of amplification reaction for each detailed performance experiment are performed.

To this end, the computer device 100 according to the present disclosure provides a feasibility tool through the DSP item to derive the results regarding whether the oligonucleotide is suitable for detecting the target nucleic acid molecule and the optimization results for the oligonucleotide. The obtained result data of amplification reaction may be provided to the feasibility tool, allowing the derived results for the oligonucleotide to be obtained from the feasibility tool.

The computer device 100 according to the present disclosure is a device used in the development process of the detection reagent for a target nucleic acid molecule, and the development of the detection reagent for a target nucleic acid molecule is based on the performance of the oligonucleotide. Therefore, it is typically important to select the oligonucleotide with the optimal performance from different candidate oligonucleotides.

Accordingly, the feasibility tool may include a selection tool used to select at least one oligonucleotide from the candidate oligonucleotides, and a performance optimization tool for optimizing the performance of the oligonucleotide selected by the selection tool.

To select the suitable oligonucleotide from the candidate oligonucleotides using the selection tool, parameters are required, which may include, for example, Ct, RFU, and parameters related to the shape of the amplification curve.

That is, the selection tool may obtain a calibrated data set from the raw data set when the result data of amplification reaction is obtained through the Data item, and may select the at least one oligonucleotide. In this case, the calibrated data set may be generated using a sigmoid function.

The performance optimization tool calibrates the result data of amplification reaction for the selected data of oligonucleotide and generates a sigmoid function. The fitting accuracy of the generated sigmoid function is determined, and using the determined fitting accuracy, the values of the signal processing parameters to be used in the presence or absence reading process of the target nucleic acid molecule may be obtained. This allows the processed/analyzed data for the selected oligonucleotide to be obtained. Through the obtaining of such processed/analyzed data, the performance of the oligonucleotide may be optimized.

Meanwhile, the computer device 100 according to the present disclosure may perform the selection of the oligonucleotide using experimental result documentation instead of the aforementioned feasibility tool.

In the at least two detailed performance experiments according to an embodiment, some experiments involve selecting the oligonucleotide to be included in the detection reagent for a target nucleic acid molecule from at least two candidate oligonucleotides.

The selection experiment may score the candidate oligonucleotides based on at least one selected among a group consisting of the sensitivity, specificity, and amplification reaction efficiency of at least two candidate oligonucleotides obtained from the result data of amplification reaction and/or processed/analyzed data received from the nucleic acid detection device 200.

In the experiment for selecting the oligonucleotide to be included in the detection reagent for a target nucleic acid molecule, when the result data of amplification reaction for each candidate oligonucleotide is received in order to select an oligonucleotide from at least two candidate oligonucleotides, respectively, the result data may be stored in the experimental result documentation of the corresponding detailed performance experiment. Simultaneously with the storage, the results required for the corresponding detailed performance experiment may be derived based on criteria predefined in the experimental result documentation. Here, since it is an experiment for selecting the oligonucleotide to be included in the detection reagent for a target nucleic acid molecule, the predefined criteria, such as sensitivity, specificity, and amplification efficiency, are extracted from the stored result data of amplification reaction. This allows for scoring the at least two candidate oligonucleotides and selecting the oligonucleotide to be included in the detection reagent for a target nucleic acid molecule.

In this case, the sensitivity and specificity of the at least two candidate oligonucleotides may be obtained from the processed/analyzed data.

The amplification reaction efficiency of the at least two candidate oligonucleotides may be obtained from the result data of amplification reaction.

Additionally, the sensitivity, specificity, and amplification reaction efficiency of the at least two candidate oligonucleotides may also be obtained from the processed/analyzed data.

The result data of amplification reaction according to an embodiment may be the raw data set received from the nucleic acid detection device 200. The raw data set includes signal intensity data according to cycles, which are expressed in terms of the number of reactions or time obtained from the signal-generation reaction for the target nucleic acid molecule using a signal-generation means. The raw data set refers to a group of unprocessed data points initially obtained from an equipment (e.g., a nucleic acid detection device), for performing a signal-generation reaction like real-time PCR.

The computer device 100 according to an embodiment extracts the values of the parameters for selection to be used for selecting at least one oligonucleotide from different candidate oligonucleotides, for each data set.

The computer device 100 according to an embodiment may generate a non-linear function fitted to such raw data sets. Generating a non-linear function refers to determining the non-linear function that best matches (or most accurately represents) the raw data set. This is basically performed by a non-linear regression analysis method.

Various conventional non-linear regression analysis methods may be used in the present invention (refer to: Seber, G. A. F.; Wild, C. J. (1989). Nonlinear Regression. New York: John Wiley and Sons). For example, polynomial functions, exponential functions, logarithmic functions, trigonometric functions, or sigmoid functions may be used as non-linear functions.

According to an embodiment of the present disclosure, the non-linear function used in the present invention is a sigmoid function. In this specification, the term "sigmoid function" refers to a function that can represent a sigmoid-shaped curve, including the logistic function, Gompertz function, or Chapman function.

In an embodiment of the present disclosure, the non-linear function may be a sigmoid function that includes at least one parameter, which is displayed in a predetermined mathematical expression. The parameters each include the parameters for the cycle number of the signal-generation reaction, the background signal value, the difference between the maximum signal value and the background signal value, the determination of the inflection point of the sigmoid function, and the determination of the sharpness of the sigmoid function. Additionally, the parameters include the parameter that integrally determines the shape of the sigmoid function, the parameter that represents the cycle at the maximum value of the second derivative function of the sigmoid function (SDM) and the ratio of the signal to the background signal value (SBR), and the slope parameter (a4) at the axis of symmetry.

The computer device 100 according to an embodiment extracts the values of the parameters for selection used for selecting the oligonucleotide from the non-linear function fitted to the raw data set, among the parameters. For example, the values of the SBR, SDM, and a4 parameters, which include parameters reflecting the shape of the amplification curve in the amplification process, may be extracted.

The computer device 100 according to an embodiment is a device used in the development process of a detection reagent for a target nucleic acid, which is performed according to a molecular diagnostic development protocol. The development of the detection reagent for the target nucleic acid is largely dependent on the performance of the oligonucleotide. Therefore, it is typically important to select the oligonucleotide with the optimal performance from the different candidate oligonucleotides.

Therefore, the computer device 100 may be provided with the values of the parameters for selection for selecting the oligonucleotide, and using these parameters for selection, may perform scoring for oligonucleotide selection through the experimental result documentation.

These experimental result documentation are electronic documents that derive and record the results required for the corresponding detailed performance experiment based on predefined criteria in at least one detailed performance experiment of the at least two detailed performance experiments.

Of course, the scoring function for the candidate oligonucleotides in the experimental result documentation needs to be preceded by the storage of the result data of amplification reaction in the experimental result documentation for each corresponding detailed performance experiment.

The scoring results, according to an example, may be displayed via a separate window in the DSP item or may be confirmed in the lab-note item.

Meanwhile, the processed/analyzed data may be a data set with the baseline subtracted from the raw data set in order to remove the background signal values. The baseline-subtracted data set may be obtained through various methods known in the art (e.g., U.S. Patent No. 8,560,247).

Meanwhile, the detailed performance experiment is a performance optimization experiment aimed at optimizing the performance of the oligonucleotide selected as the oligonucleotide to be included in the detection reagent for a target nucleic acid molecule from the at least two candidate oligonucleotides, and may include at least one of the following: sensitivity and specificity when the amplification composition including the selected oligonucleotide is used to detect target nucleic acid molecules of different concentrations; sensitivity and specificity for either the standard strain that needs to be detected by the amplification composition including the selected oligonucleotide or the excluded strain that needs to be excluded from detection; sensitivity and specificity in case that the amplification composition including the selected oligonucleotide is accommodated in different accommodation vessel types and used for detection; sensitivity and specificity in case that only a single target nucleic acid molecule is included, or at least two or more target nucleic acid molecules are accommodated together in a reaction vessel in which a amplification composition including the selected oligonucleotide is accommodated, wherein the amplification composition including the selected oligonucleotide is used for analyzing at least two target nucleic acid molecules, sensitivity and specificity for either clinical samples including the target nucleic acid molecule to be detected by the amplification composition including the selected oligonucleotide or clinical samples not including the target nucleic acid molecule.

According to an embodiment, at least one of the data set for the selected oligonucleotide and the values of the parameters for selection extracted for the selected oligonucleotide may be converted into a preset documentation format and stored in the experimental result documentation. Before converting the data set for the selected oligonucleotide and the values of the parameters for selection extracted for the selected oligonucleotide into the preset documentation format, performance analysis of the raw data set for the selected oligonucleotide may be additionally performed.

To this end, the computer device 100 includes an algorithm for calibrating the raw data set for the selected oligonucleotide. For example, the computer device 100 includes the algorithm that applies a normalization coefficient, as disclosed in the applicant's WO 2017/086762, to the signal values to obtain the calibrated data.

The normalization coefficient is provided using a reference value, a reference cycle, and the data set. The reference cycle is selected among the cycles of the data set, the reference value is an arbitrarily set value, and the normalization coefficient is provided by establishing a relationship between the signal value of the cycle corresponding to the reference cycle in the data set and the reference value. Further, the normalization coefficient is then applied to the signal values of the raw data set to obtain the calibrated signal values, which are provided to the raw data set. An example is the reference value. The reference value is the value used to provide the normalization coefficient. In this specification, the reference value refers to any value applied to the reference cycle in order to calibrate the signal values of the data set. The same reference value is applied to the data sets that are to be normalized according to the same criteria. When the data sets to be calibrated are at least two data sets, the at least two data sets may be calibrated by the same reference value. The reference value may be an arbitrarily set value. Preferably, the reference value is an arbitrarily set value among non-zero real numbers. The reference value may be arbitrarily selected by the experimenter, as long as the presence of the target analyzed substance in the sample may be determined by the correction data set using the reference value. Therefore, the reference value used for the correction of the data set may be determined within the range of signal values that may be obtained at the reference cycle by the same type of signal-generation reaction from which the data set was obtained. The reference value may be obtained separately from the data set to be calibrated. Specifically, the reference value may be determined from a data set obtained from a signal-generation reaction for a target nucleic acid molecule of the same type as the target analyzed substance being analyzed. Alternatively, the reference value may be obtained from a group of data sets that includes the data set to be calibrated. Preferably, the reference value may be a value of the same type as the signal values of the data set to be calibrated, and may have the same unit or dimension as the data set to be calibrated. However, even if the reference value and the signal values of the data set have different units or dimensions, or even if the reference value does not have any units or dimensions, the suitable normalization coefficient for each reaction may be provided from the reference value and the signal values of the data set to be calibrated. The correction data set may then be obtained using the normalization coefficient for each reaction. The parameters may be distinguished based on the type of analysis method and classified and displayed according to their type. Accordingly, the preset default values may also be distinguished by parameter and displayed according to the type of analysis method.

These parameters are used either for the mathematical processing of the signal in a signal generation reaction that generates a signal dependent on the presence of the target nucleic acid molecule, or as a criterion for the presence or absence reading of the target analyzed substance in the sample. Examples of values that may be used to determine the positive/negative of a sample include Ct (Threshold Cycle), delta Ct, melt Tm, melt peak, the threshold intensity value of the signal used for determining the Ct, the threshold value used to determine the Ct value, the fitting start cycle and the final cycle used for background subtraction; the reference value and reference cycle when adopting the algorithm disclosed in WO 2017/086762; the R² value, the maximum slope value of the sigmoid fitting curve, and the difference between the maximum and minimum signal values when using the sigmoid fitting algorithm; and the reference value when adopting the algorithms disclosed in WO 2015-147370, WO 2015/147412, WO 2015/147382, and WO 2016/093619.

Therefore, the BPN of the analysis method may include parameters for the reference value (RV) and the signal value-related parameter (Cut-off ratio (CR)) value. The CR describes the relationship or degree of signal variation, or signal difference when two signal values occur at different detection temperatures, particularly in numerical terms. In this case, the different detection temperatures may be a relatively high detection temperature and a relatively low detection temperature, or a first temperature and a second temperature. The "signal value-related parameter" includes any value that reflects the pattern (rule) of signal variation at different detection temperatures. In addition, the signal value-related parameter refers to a value that indicates the degree of change between the signal detected at a relatively high detection temperature and the signal detected at a relatively low detection temperature for a specific target nucleic acid sequence. The signal value-related parameter may refer to a value used to convert, transition, adjust, or modify the signal detected at one temperature to the signal at another temperature. The signal value-related parameter may vary depending on the type of target nucleic acid sequence, the type of signal generation means, and the conditions of incubation and detection. Therefore, various signal value-related parameters may be determined for different or the same target nucleic acid sequences.

The presence or absence reading process of the target nucleic acid molecule refers to reading whether the target nucleic acid molecule is present in the sample, based on the data set obtained from the nucleic acid detection device 200. The presence or absence reading process according to the present disclosure is used to read whether each sample is positive or negative, using the data sets obtained from each sample that provide ground truth data (data in which the presence/absence result of the target nucleic acid molecule has been confirmed). To read the positive/negative, parameters are required. For example, the parameters used in the presence or absence reading process may include the threshold intensity value of the signal used to determine the Ct (Threshold Cycle) for each sample as a criterion for determining the presence or absence of the target nucleic acid molecule in the sample. Alternatively, the parameters used in the presence or absence reading process may include parameters used in the processed performance data set, which is obtained by mathematically processing the raw data set.

The computer device 100 according to an embodiment may provide a positive/negative reading criterion that may set the value for the Ct parameter to be used in the presence or absence reading process of the target nucleic acid molecule in the sample. Alternatively, in accordance with an embodiment, the computer device 100 may perform analysis for the presence or absence reading process of the target nucleic acid molecule using the DSP (WO2019/066572) technology as the positive/negative reading criterion. The parameters set using the DSP technology include parameters used in the processed performance data set, which is obtained by mathematically processing the raw data set. Here, the processed/analyzed data not only includes the data obtained by mathematically processing the raw data set but also includes the data obtained by further mathematically processing the mathematically processed data that has been obtained in this way. For example, the processed/analyzed data includes data such as a first derivative of the raw data set and the derivatives such as second, third, and higher-order derivatives that area obtained from the first derivative.

In the DSP item, the fitting accuracy of the non-linear function for the data set is used as a direct indicator for the analysis of the target nucleic acid molecule. This involves obtaining a data set for the target nucleic acid molecule from a signal-generation reaction that uses a signal-generation means, to analyze the target analyzed substance without false-positive or false-negative results, in particular false-positive results, calibrating the data set, which includes at least two data points including cycle numbers and signal values, determining the fitting accuracy of a non-linear function for the calibrated data set by generating the non-linear function for the calibrated data set, and determining the presence or absence of the target analyte in the sample using the fitting accuracy. This analysis method requires various parameters, and optimization may be performed based on the values set for each parameter. The DSP (WO2019/066572) technology is used to assign parameters used in the presence or absence reading process of the target analyzed substance, and analysis is performed by setting the values of the DSP parameters.

Additionally, an algorithm for extracting only data for each target analyzed substance from data obtained at at least two different temperatures for at least two target nucleic acid molecules may be included. For example, using the algorithms disclosed in the applicant's WO2015-147370, WO2015/147412, WO2015/147382, and WO2016/093619, which uses at least two target detection temperatures, the parameters used in the presence or absence reading process of the target analyzed substance may be assigned. By setting the values of the assigned parameters, the data of the raw data set may be analyzed.

In the DSP item for each at least two detailed performance experiment, the selected oligonucleotide is optimized by confirming the results (positive/negative read results) or changes (amplification curves) by varying the setting values and analyzing the outcomes. In this case, in this process, the default value and the setting value are displayed on a single screen to allow the results of the positive/negative reading analyzed based on the preset reference setting values, for example, the default value, to be simultaneously confirmed or compared with the setting values set by the researcher or developer.

The value of the optimal parameter needs to be analyzable to match the information for each well for positive/negative labeling in the ground truth data under any conditions and environment.

In the present disclosure, the data sets of the amplification process results obtained through performance experiments under different experimental conditions and environments are each obtained for the selected oligonucleotide. By repeatedly adjusting the values of the parameters for the selected oligonucleotide, the optimal parameter values may be set until the positive is confirmed as positive and the negative as negative in the ground truth data, using the display of the default value and setting value.

In the DSP item according to the present disclosure, processing/analyzing the received result data of the amplification reaction to obtain processed/analyzed data the received result data of the amplification reaction for at least two detailed performance experiments may be may be processed/analyzed to obtain processed/analyzed data. Here, wherein the processing/analyzing of the received result data of the amplification reaction to obtain the processed/analyzed data may be performed via a setup screen and a default screen, wherein the setup screen sets up setting values used for processing/analysis in at least one of the at least two detailed performance experiments, and displays results where the setting values set up are applied to, wherein in at least one of detailed performance experiments, the default screen displays results where setting values pre-set up via the setup screen are applied to ,

Also, wherein the setup screen and the default screen may be displayed on a single screen.

In the present disclosure, wherein the obtaining of the processed/analyzed data may include processing the received result data of the amplification reaction to obtain a positive/negative read data to be used for a presence or absence reading process of the target nucleic acid molecule, and is performed through parameters for correction and parameters for numerical adjustment with respect to the received result data of the amplification reaction.

Wherein the parameters for correction may include a parameter criterion (PMC), which relates to determining a shape of a sigmoid graph for the received result data of the amplification reaction, a start fitting cycle (SFC), for adjusting the starting cycle for baseline fitting and

Wherein the parameters for numerical adjustment may include a cut-off ratio (CR), which is a value obtained by dividing a RFU value at low temperature by a RFU value at high temperature to calibrate a difference in REU values due to the difference in RFU values at low/high temperatures, a THRD (Ct Threshold), which is a threshold value for determining the positive/negative result reading of a sample after a sigmoid fitting and a dRFU (determinant RFU), which is used to filter positive/negative determination result of a sample.

The research and development data derived through this process, that is, the data set for the selected oligonucleotide (the raw data set and the calibrated data set) or the parameters (e.g., parameters reflecting the shape of the amplification curve in the amplification process from the raw data set and the Ct and End-RFU from the calibrated data set), are obtained and stored in the experimental result documentation.

Moreover, the present disclosure is not limited to the operation of research and development data for the selected oligonucleotide. In the selection tool, during the process of selecting the suitable oligonucleotide from the candidate oligonucleotides, the data set or parameters for the oligonucleotides that were not selected may be stored in the lab-note. The computer device 100 according to an embodiment may store the data input via the data input screen for the requirement items for performing an experiment, the preparation information, the amplification reaction result, and the processed/analyzed data as an experimental result documentation for each detailed performance experiment.

According to an embodiment, the storing into the experiment result record sheet for each detailed performance experiment comprises collecting predefined results for each step and organizing the collected predefined results according to a preset format for each detailed performance experiment.

Therefore, the lab-note item corresponding to the experimental result documentation generates experimental result documentation for at least two performance experiments.

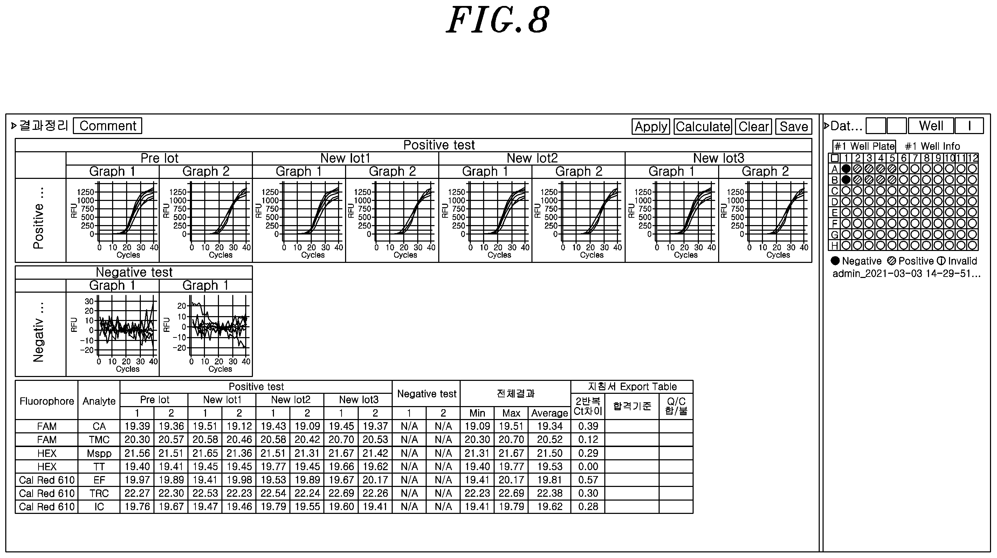

FIG. 8 is an exemplified view illustrating the form of the experimental result documentation generated in the computer device 100 according to an embodiment.

As illustrated in FIG. 8, the experimental result documentation are where the research and development data derived during the research and development process is recorded, and are a record of at least one requirement item for performing an experiment selected among the requirement items for performing an experiment (item for experimentation) that include the materials used for each detailed performance experiment for the research and development of the detection reagent for a target nucleic acid molecule, methods for the experiments, experimental devices, and a combination thereof, preparation information, amplification reaction results, and processed/analyzed data.

According to an embodiment, the lab-note is generated for each of the at least two detailed performance experiments, as illustrated in FIG. 5, based on the information input via the data input screens in FIG. 3 and FIG. 4.

As described above, each detailed performance experiment includes detailed procedures, that is, Material & Method, well info, Data, DSP, Lab-note, and Setting value items. Therefore, the information input via the data input screen for each item and the software operation results for each item may all be recorded in the lab-note.

For example, in the experimental result documentation for the Multi MOM performance_Co_infection; Channerl performance validation experiment, the following may be recorded: information input through the Material & Method item and an image of the plate on which the preparation information automatically generated based on this information through the Well info item is displayed; information on the equipment on which the Multi MOM performance_Co_infection; Channerl experiment was conducted and the data set file information obtained from this information through the Data item; History information that determines the oligonucleotide selected among the candidate oligonucleotides through the DSP item; and history information for optimizing the performance of the selected oligonucleotide, and optimization results.

Since the computer device 100 according to the present disclosure is used to process, manage, and store data calculated according to a molecular diagnostic development protocol during the development process of the target nucleic acid molecule, it is preferable to store the history of each process of the reagent development according to the molecular diagnostic development protocol.

Therefore, the computer device 100, as described, is not limited to the operation of data for the selected oligonucleotide only. All data calculated in the process of developing a detection reagent for the target analyzed substance reflects the characteristics of the reagent development. Therefore, the data set or parameters in the specific experiments for the candidate oligonucleotides that were not selected among the candidate oligonucleotides may be additionally recorded in the experimental result documentation.

Further, the Setting value item automatically stores the setting values applied to the experimental result documentation for each detailed performance experiment of the selected oligonucleotide.

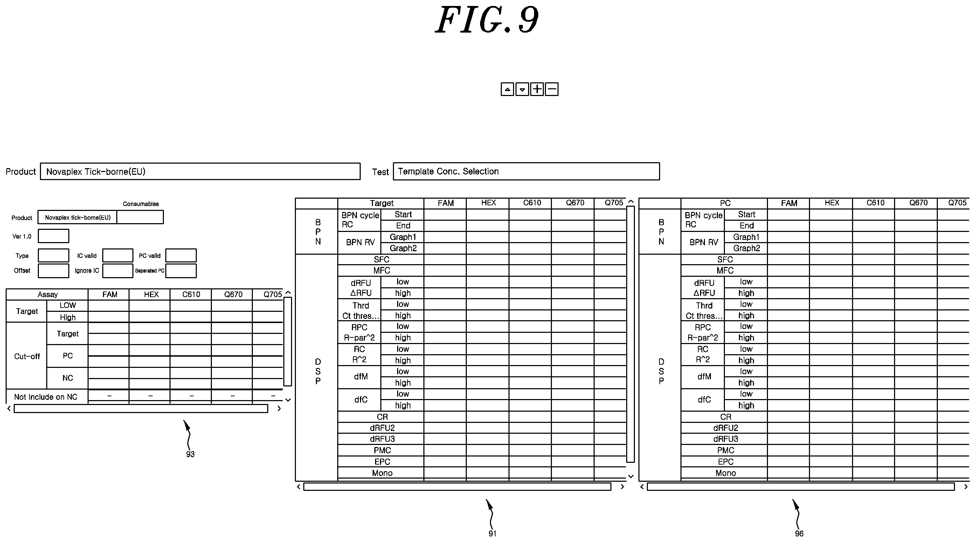

FIG. 9 is an exemplified view illustrating the Setting value display screen in the computer device 100 according to an embodiment.

With reference to FIG. 9, through the Setting value item, the computer device 100 according to the present disclosure may allow a default value 91 and a setting value 92 to be displayed together for comparison at once in the corresponding detailed performance experiment to the researcher or developer who uses the Setting value item.

In the Setting value item, the default value and setting value are displayed selectively for each detection channel.

To this end, the computer device 100 according to the present disclosure may arrange and display the detection channels in a table 93, allowing for selecting a detection channel.

That is, in the Setting value item, for each detection of at least two target nucleic acid molecules (low temperature/high temperature) with a single channel, and/or for detection of at least two target nucleic acid molecules with at least two channels, the default value and setting value for each of these at least two target nucleic acid molecules may be arranged and displayed.

In this way, through the Setting value item, the researcher or developer may confirm the presence or absence read setting values for the default value and setting values applied for each detailed performance experiment. Not only can the researcher or developer quickly and accurately set the values of parameters by referring to the confirmed setting values, but they can also apply various setting values for each experiment for performance validation.

Meanwhile, the computer device 100 according to the present disclosure, as described, automatically stored setting values via the Setting value item may be set by providing a setup screen for setting up a setting value for data processing used for processing of the aforementioned result data of amplification reaction and/or a setting value for presence or absence reading used for presence or absence reading of the target nucleic acid molecule in the sample. In this case, the computer management device 100 according to the present disclosure matches the setting values for processing and/or the setting values for presence or absence reading input via the setup screen with the meta-data.

The setting values for processing and/or the setting values for presence or absence reading, matched with the meta-data, are stored in a predetermined position within the data storage structure in a structured format.

Additionally, the computer device 100 according to the present disclosure, upon obtaining result data of amplification reaction for the target nucleic acid molecule, which is one of results of at least two detailed performance experiments, matches the obtained result data of amplification reaction with the meta-data, and stores the result data of amplification reaction, matched with the meta-data, in a predetermined position within the data storage structure. The result data of amplification reaction in this case is also stored in a structured format. For example, in the result data of amplification reaction, the signal intensity, which is a signal value at a specific cycle, needs to be input in units of RFU, and needs to be input for each cycle. For the amplification graph, the graph needs to be displayed with pre-designated colors for each channel, and the scale of the amplification graph may be normalized.

Through the DSP item, when the amplification result data, which is the results processed by the setting value for processing and/or the presence or absence read results for the target nucleic acid molecule, with the setting value for presence or absence reading applied, are obtained, the processed results and/or the presence or absence read results are matched with the meta-data. The processed results and/or the presence or absence read results, matched with the meta-data, are then stored in a predetermined position within the data storage structure.

The meta-data is data for the data stored in a predetermined position within a pre-designed data storage structure. According to an embodiment, various data derived during the research and development process, for example, data input via the data input screens (screens illustrated in FIG. 3 to FIG. 7 and items thereof (Data and DSP item)) is each stored as meta-data in a structured format. The data input in a non-structured format may be converted into a structured format, or the input itself may be rejected.

The data storage structure in the computer device 100, where the meta-data is stored, is a structure in which each research and development data derived during the research and development process of the detection reagent for a target nucleic acid molecule is indexed as meta-data and structured based on the target nucleic acid molecule of the detection reagent for a target nucleic acid molecule to be developed. For example, in the data storage structure, the target analyzed substance for the detection reagent for the target analyzed substance becomes a root node, and each piece of research and development data derived during the research and development process of the reagent becomes a child node. In the data storage structure, the root node has at least one child node.

The child nodes are meta-data indexed for each piece of research and development data derived during the research and development process. One child node is connected to at least one other child node, and at least one piece of individual data is accumulated in each child node.

Each individual piece of data accumulated in the child node may be a test that the researcher or developer has tried in various ways. For example, in the meta-data indexed by the setting value, setting values tested by a single researcher or developer at least two times and/or setting values tested by each researcher or developer to setup the optimized setting values for the development of a specific target analyzed substance detection reagent, are all accumulated and stored in the indexed setting value meta-data. This setting value meta-data stores both the setting values that were actually applied to the reagent and those that were not applied.

The indexed meta-data may include amplification reaction results, setting values, parameters used in the presence or absence reading process, at least two performance validation experiments, experiment information (type, materials, procedures, equipment, etc.), product names, and viewer results. These are stored respectively in predetermined positions within the pre-designed data storage structure.

To this end, the computer device 100 according to the present disclosure controls, by software, the data input via the data input screen to be stored in a structured format at the pre-designated positions (e.g., the corresponding indexed meta-data positions) within the pre-designed data storage structure. That is, the research and development data management device 100 may control, through computer-readable codes or instructions stored on a computer-readable recording medium executable on the processor 130, the input data to be stored in a predefined position within the data storage structure.

This allows the data to be searchable by organizing by type of various types of research and development data derived from the start to the result of the various experiments performed during the research and development process, and by storing them in a database.

As a result, when a researcher or developer using the computer device 100 aims to develop a specific detection reagent for a target nucleic acid molecule or optimize the performance of the oligonucleotide for a specific reagent for detecting a target nucleic acid molecule, this allows for the reuse or search of research and development data derived during the research and development process for each detection reagent for a target nucleic acid molecule.

The computer device 100 according to the present disclosure may search for the meta-data related to a query by using a instruction that returns the meta-data as the corresponding query.

Accordingly, the computer device 100 provides a search condition input screen, and receives the search conditions via the input screen. The meta-data matching the received search conditions is searched from the meta-data stored in a predetermined position within the data storage structure. The searched meta-data and/or the amplification result data matching the searched meta-data are obtained.

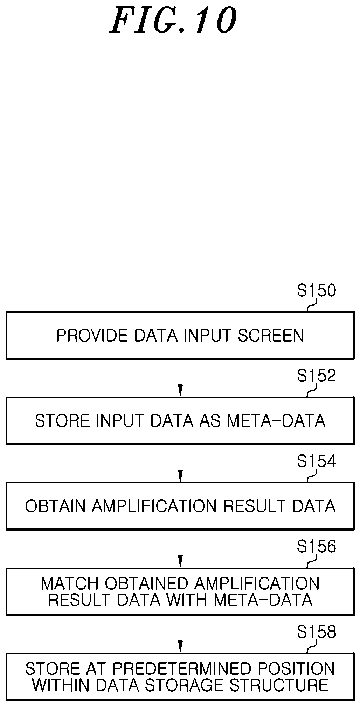

FIG. 10 is a flowchart illustrating a method of managing research and development data in the research and development data management device 100 according to an embodiment.

With reference to FIG. 10, in S150, the computer device 100 provides a data input screen for at least one requirement item for performing an experiment selected among the requirement items for performing an experiment (item for experimentation) that include materials, experimental procedures, experimental devices, and a combination thereof, to be used in the detailed performance experiment performed on the candidate oligonucleotide.

In S152, the data input via the data input screen is stored as meta-data in a predetermined position within the pre-designed data storage structure.

In S154, the computer device 100 obtains the result data of amplification reaction for the target nucleic acid molecule, which is one of the results of the detailed performance experiment.

In S156, the obtained result data of amplification reaction is matched with the meta-data.

In S158, the result data of amplification reaction, matched with the meta-data, is stored in a predetermined position within the data storage structure.

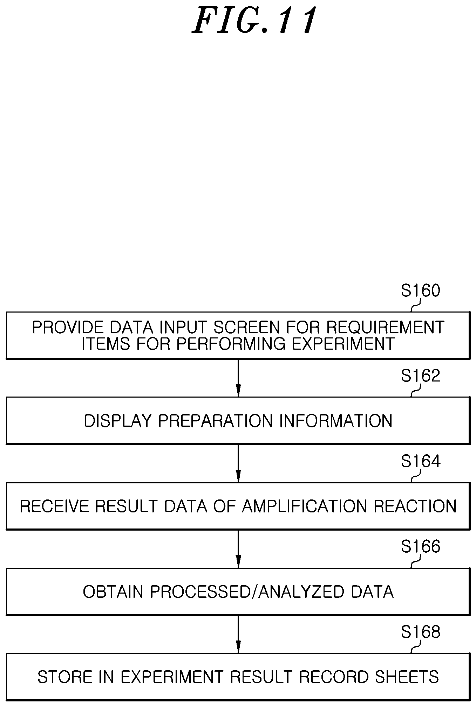

FIG. 11 is a flowchart illustrating a method of guiding at least two detailed performance experiments for the development of a detection reagent for a target nucleic acid molecule and organizing experimental result documentation in the computer device 100 according to an embodiment.

In S160, the computer device 100 provides a data input screen for at least one requirement item for performing an experiment selected among the requirement items for performing an experiment (item for experimentation), which include materials, experimental procedures, experimental devices, and a combination thereof, to be used for each of the detailed performance experiments performed on the oligonucleotide for detecting the target nucleic acid molecule.

In S162, based on the data input via the data input screen for the requirement items for performing an experiment for each detailed performance experiment, the preparation information is displayed for the reaction plate, which is used by the nucleic acid detection device to perform the amplification reaction on the oligonucleotide.

In S164, based on the preparation information, the result data of amplification reaction performed by the nucleic acid detection device is received using the amplification composition including the oligonucleotide prepared according to the preparation information for each detailed performance experiment.

In S166, the received result data of amplification reaction is processed/analyzed to obtain the processed/analyzed data.

In S168, the data input via the data input screen for the requirement items for performing an experiment, the preparation information, the amplification reaction result, and the processed/analyzed data are stored in the experimental result documentation for each detailed performance experiment.

The operations at each step illustrated in FIG. 10 and FIG. 11 are the same as the functions of the computer device 100 described above. Therefore, the descriptions of FIG. 1 to FIG. 9 are incorporated by reference.

As described above, the method of managing research and development data performed in the computer device 100 according to the present disclosure involves indexing and storing the research and development data derived during the research and development process as meta-data in a structured data storage structure. Based on the stored research and development data, a lab-note may be completed. The researcher or developer may perform experiment planning, experimental procedures, and experimental result analysis and organization through the research and development data management device 100. Based on the meta-data stored in the data storage structure, they can reuse or search for analysis information on experimental results (e.g., oligonucleotide selection and optimization, and the history information that determines these).

The block diagrams and the combination of each block with the steps of the flowcharts attached to this disclosure may be performed by a computer program.

The computer program, when executed by one or more processors, includes instructions to cause the one or more processors to perform a method of managing the research and development data of a candidate oligonucleotide for detecting a target analyzed substance in a research and development data management device. The method includes the steps of: providing a data input screen for at least one group selected among a group that includes types of performance validation experiments to be performed on the candidate oligonucleotide, materials used in the experiments, the experimental procedures in the experiments, the experimental devices, and a combination thereof;
storing data input via the data input screen as meta-data in a predetermined position within a pre-designed data storage structure; obtaining result data of amplification reaction for the target analyzed substance, which is one of results of the performance validation experiment; matching the obtained result data of amplification reaction with the meta-data; and storing the result data of amplification reaction, matched with the meta-data, in a predetermined position within the data storage structure.

In the present disclosure of a computer device comprising a memory storing at least one instruction; a processor; and wherein the at least one instruction, when executed by the processor, causes the computer device to: provide a data input screen for at least one item for experimentation selected among items for experimentation, to be used in performing each detailed performance experiment on an oligonucleotide for detecting the target nucleic acid molecule, wherein the items include materials, experimental procedures, experimental devices, and a combination thereof, the items for experimentation being for a use in performing each detailed performance experiment on an oligonucleotide for detecting the target nucleic acid molecule; display preparation information for a reaction plate, to be used for a nucleic acid detection device to perform an amplification reaction using the oligonucleotide, based on data input via the data input screen for the item for experimentation for each detailed performance experiment; receive result data of the amplification reaction performed by the nucleic acid detection device, using an amplification composition including the oligonucleotide prepared according to the preparation information for each detailed performance experiment; process/analyze the received result data of the amplification reaction to obtain processed/analyzed data; and store the data input via the data input screen for the item for experimentation, the preparation information, the result of the amplification reaction, and the processed/analyzed data as an experimental result documentation for each detailed performance experiment.

A computer program stored in a non-transitory computer-readable storage medium, wherein the computer program, when executed by at least one processor included in a computer device, causes the computer device to perform a method including:
providing a data input screen for at least one item for experimentation selected among items for experimentation, to be used in performing each detailed performance experiment on an oligonucleotide for detecting the target nucleic acid molecule, wherein the items include materials, experimental procedures, experimental devices, and a combination thereof,; displaying preparation information for a reaction plate, to be used a nucleic acid detection device to perform an amplification reaction using the oligonucleotide, based on data input via the data input screen for the item for experimentation for each detailed performance experiment;
receiving result data of the amplification reaction performed by the nucleic acid detection device, using an amplification composition including the oligonucleotide prepared according to the preparation information for each detailed performance experiment; processing/analyzing the received result data of the amplification reaction to obtain processed/analyzed data; and storing the data input via the data input screen for the item for experimentation, the preparation information, the result of the amplification reaction, and the processed/analyzed data as an experimental result documentation for each detailed performance experiment.

A non-transitory computer-readable storage medium storing a computer program, wherein the computer program, when executed by at least one processor included in a computer device, causes the computer device to perform a method including:
providing a data input screen for at least one item for experimentation selected among items for experimentation, to be used in performing each detailed performance experiment on an oligonucleotide for detecting the target nucleic acid molecule, wherein the items include materials, experimental procedures, experimental devices, and a combination thereof, the items for experimentation being for a use in performing each detailed performance experiment on an oligonucleotide for detecting the target nucleic acid molecule;
displaying preparation information for a reaction plate, to be used for a nucleic acid detection device to perform an amplification reaction using the oligonucleotide, based on data input via the data input screen for the item for experimentation for each detailed performance experiment; receiving result data of the amplification reaction performed by the nucleic acid detection device, using an amplification composition including the oligonucleotide prepared according to the preparation information for each detailed performance experiment; processing/analyzing the received result data of the amplification reaction to obtain processed/analyzed data; and storing the data input via the data input screen for the item for experimentation, the preparation information, the result of the amplification reaction, and the processed/analyzed data as an experimental result documentation for each detailed performance experiment.

Such a program may also be stored in a non-transitory computer-readable storage medium to implement functions in a specific manner. In addition, unless explicitly stated otherwise, the terms 'include,' 'comprise,' or 'contain' described above should be understood to mean that the relevant components may be inherently included, and thus should not be interpreted as excluding other components but as allowing the inclusion of additional components. All terms, including technical or scientific terms, shall have the same meaning as generally understood by those of ordinary skill in the art to which the present disclosure pertains, unless otherwise defined. Commonly used terms, such as those defined in dictionaries, shall be interpreted consistently with their contextual meaning in the relevant technical field and shall not be construed in an idealized or unduly formal sense unless explicitly defined in the present disclosure.

The above description is merely exemplary description of the technical scope of the present disclosure, and it will be understood by those skilled in the art that various changes and modifications can be made without departing from original characteristics of the present disclosure. Therefore, the embodiments disclosed in the present disclosure are intended to explain, not to limit, the technical scope of the present disclosure, and the technical scope of the present disclosure is not limited by the embodiments. The protection scope of the present disclosure should be interpreted based on the following claims and it should be appreciated that all technical scopes included within a range equivalent thereto are included in the protection scope of the present disclosure.

As described above, specific portions of the present disclosure have been detailed, but it is apparent that such specific descriptions are merely preferred embodiments for those of ordinary skill in the art and do not limit the scope of the present disclosure. Accordingly, the substantive scope of the present disclosure shall be defined by the appended claims and their equivalents.

## Claims

1. A method for guiding at least two detailed performance experiments required for development of a detection reagent for a target nucleic acid molecule and organizing an experimental result documentation, to be performed by a computer device, the method comprising:
providing a data input screen for at least one item for experimentation selected among items for experimentation to be used in performing each detailed performance experiment on an oligonucleotide for detecting the target nucleic acid molecule, wherein the items include materials, experimental procedures, experimental devices and a combination thereof;
displaying preparation information for a reaction plate to be used for a nucleic acid detection device to perform an amplification reaction using the oligonucleotide, based on data input via the data input screen for the items for experimentation for each detailed performance experiment;
receiving result data of the amplification reaction performed by the nucleic acid detection device using an amplification composition including the oligonucleotide prepared according to the preparation information for each detailed performance experiment;
processing/analyzing the received result data of the amplification reaction to obtain processed/analyzed data; and
storing the data input via the data input screen for the item for experimentation, the preparation information, the result of the amplification reaction, and the processed/analyzed data as an experimental result documentation for each detailed performance experiment.

2. The method of claim 1, further comprising:
displaying a detailed performance experiment input screen for selecting types of the detailed performance experiments,
wherein each of the detailed performance experiments is for testing performance of the oligonucleotide, and the performance of the oligonucleotide includes sensitivity and/or specificity for the target nucleic acid molecule.

3. The method of claim 2, wherein the detailed performance experiments are experiments for selecting an oligonucleotide to be included in the detection reagent for the target nucleic acid molecule from at least two candidate oligonucleotides, and
wherein the experiments for selecting comprises:
scoring the candidate oligonucleotides based on at least one selected among a group consisting of sensitivity, specificity, and amplification reaction efficiency of the at least two candidate oligonucleotides, obtained from the received result data of the amplification reaction and/or the processed/analyzed data.

4. The method of claim 3, wherein the sensitivity and specificity of the at least two candidate oligonucleotides are obtained from the processed/analyzed data, and the amplification reaction efficiency of the at least two candidate oligonucleotides are obtained from the amplification reaction result.

5. The method of claim 3, wherein the sensitivity, the specificity, and the amplification reaction efficiency of the at least two candidate oligonucleotides are obtained from the processed/analyzed data.

6. The method of claim 1, further comprising:
providing a shared data input screen for at least one item for experimentation selected among items for experimentation to be used in performing the at least two detailed performance experiments,
wherein the items include materials, experimental procedures, experimental devices and a combination thereof, at least two detailed performance experiments,
wherein a shared data input via the shared data input screen is provided according to a user's selection as an input data to the data input screen for the item for experimentation for each detailed performance experiment.

7. The method of claim 6, wherein at least one experimental materials-related data input in the shared data input screen is selected among a group consisting of type and concentration of an oligonucleotide, type and concentration of a target nucleic acid molecule, a type of enzyme, buffer, a type of a reaction vessel, type and concentration of positive control, type and concentration of negative control, type and concentration of internal control, type and amount in use of water, and a combination thereof.

8. The method of claim 6, wherein at least one experimental procedures-related data input in the shared data input screen is selected among a group consisting of information on a target nucleic acid molecule for each detection channel, an amplification reaction protocol, an extraction method of the target nucleic acid molecule, and a combination thereof.

9. The method of claim 6, wherein at least one experimental procedures-related data input in the shared data input screen is selected among a group consisting of a nucleic acid detection device, an experimental preparation device, and a combination thereof.

10. The method of claim 1, wherein the step of displaying the preparation information comprises:
displaying at least one selected from the group consisting of a type of a template and/or a concentration or series of concentrations of the template, a type of an oligonucleotide, and a type of a reaction plate, each of which is suitable for a purpose of the detailed performance experiments.

11. The method of claim 10, wherein the type of the template and/or the concentration or the series of concentrations of the template is displayed in each of at least two reaction vessels included in the reaction plate.

12. The method of claim 1, further comprising:
storing the data input via the data input screen as a meta-data at a predetermined position within a pre-designed data storage structure;
matching the received result data of the amplification reaction and the processed/analyzed data with the meta-data; and
storing the result data of the amplification reaction and processed/analyzed data matched with the meta-data at a predetermined position within the data storage structure.

13. The method of claim 12, further comprising:
providing a setup screen for setting up setting values used in processing/analyzing the result data of the amplification reaction;
matching the setting values input via the setup screen with the meta-data; and
storing the setting values matched with the meta-data at a predetermined position within the data storage structure.

14. The method of claim 1, further comprising:
providing a search condition input screen;
receiving search conditions via the search condition input screen;
searching for meta-data that matches the received search conditions from meta-data stored at a predetermined position within the data storage structure; and
obtaining the searched meta-data and/or result data of the amplification reaction matched with the searched meta-data.

15. The method of claim 1, wherein the processing/analyzing of the received result data of the amplification reaction to obtain the processed/analyzed data is performed via a setup screen and a default screen,
wherein the setup screen sets up setting values used for processing/analysis in at least one of the at least two detailed performance experiments and displays results where the setting values set up are applied to,
wherein in at least one of detailed performance experiments, the default screen displays results where setting values pre-set up via the setup screen are applied to, and
wherein the setup screen and the default screen are displayed on a single screen.

16. The method of claim 1, wherein the storing as the experimental result documentation for each detailed performance experiment comprises:
collecting predefined results for each step; and
organizing the collected predefined results according to a format pre-designated for each detailed performance experiment.

17. The method of claim 16, wherein the experimental result documentation is an electronic document that is, based on predefined criteria in at least one detailed performance experiment of the at least two detailed performance experiments, derived and recorded results required for the detailed performance experiment from the collected predefined results.

18. The method of claim 2, wherein the at least two detailed performance experiments are performance optimization experiments to optimize performance of an oligonucleotide selected from at least two candidate oligonucleotides as an oligonucleotide to be included in the detection reagent for the target nucleic acid molecule, and
wherein the at least two detailed performance experiments include at least one of:
sensitivity or specificity in case that an amplification composition including the selected oligonucleotide is used to detect target nucleic acid molecules of different concentrations;
sensitivity and specificity for either a standard strain that needs to be detected by a amplification composition including the selected oligonucleotide or an excluded strain that needs to be excluded from detection;
sensitivity and specificity in case that a amplification composition including the selected oligonucleotide is accommodated in different type of accommodation vessels and used for detection;
sensitivity and specificity in case that only a single target nucleic acid molecule is included, or at least two or more target nucleic acid molecules are accommodated together in a reaction vessel in which a amplification composition including the selected oligonucleotide is accommodated, wherein the amplification composition including the selected oligonucleotide is used for analyzing at least two target nucleic acid molecules,
sensitivity and specificity for either clinical samples including a target nucleic acid molecule to be detected by a amplification composition including the selected oligonucleotide or clinical samples not including a target nucleic acid molecule.

19. The method of claim 2, wherein the step of displaying the detailed performance experiment input screen for selecting types of the at least two detailed performance experiments comprises:
providing a section that displays a progress status as an icon for each of the items for experimentation for each detailed performance experiment, the preparation information, the result data of the amplification reaction, the processed/analyzed data and the experimental result documentation
wherein, selection the icon displays a screen showing detailed information of the corresponding the items for experimentation, the preparation information, the result data of the amplification reaction, the processed/analyzed data, or the experimental result documentation.

20. The method of claim 1, further comprising:
providing a parameter input screen for inputting values of parameters for each manufacturing task, the values of parameters being required for driving an experimental preparation device preparing the amplification composition used for each of the detailed performance experiments,
wherein the manufacturing tasks include at least one of:
a detailed task for a template dilution used in an amplification reaction;
a detailed task for an oligonucleotide mixture;
a detailed task for a master mixture including enzyme, buffer, and the oligonucleotide mixture; and
a detailed task for setting up an amplification reaction solution by dispensing the master mixture and the template dilution into a reaction vessel.

21. The method of claim 1, wherein the step of obtaining the processed/analyzed data is performed using parameters for correction and parameters for numerical adjustment with respect to the received result data of the amplification reaction,
wherein the parameters for correction include:
a parameter criterion (PMC), for determining a shape of a sigmoid graph for the received result data of the amplification reaction;
a start fitting cycle (SFC), for adjusting the starting cycle for baseline fitting correction; and
a minimum fitting cycle (MFC), for correcting the result data of the amplification reaction by performing baseline fitting from the SFC (Start Fitting Cycle) to the MFC (Minimum Fitting Cycle),
wherein the parameters for numerical adjustment include:
a cut-off ratio (CR), which is a value obtained by dividing a RFU value at low temperature by a RFU value at a high temperature to calibrate a difference in REU values caused by the difference in RFU values between the low/high temperatures;
a THRD (Ct Threshold), which is a threshold value for determining the positive/negative determination result of a sample after a sigmoid fitting; and
a dRFU (determinant RFU), which is used to filter positive/negative determination result of a sample.

22. A computer device comprising:
a memory storing at least one instruction;
a processor; and
wherein the at least one instruction, when executed by the processor, causes the computer device to:
provide a data input screen for at least one item for experimentation selected among items for experimentation, to be used in performing each detailed performance experiment on an oligonucleotide for detecting the target nucleic acid molecule, wherein the items include materials, experimental procedures, experimental devices, and a combination thereof, the items for experimentation being for a use in performing each detailed performance experiment on an oligonucleotide for detecting the target nucleic acid molecule;
display preparation information for a reaction plate, to be used for a nucleic acid detection device to perform an amplification reaction using the oligonucleotide, based on data input via the data input screen for the item for experimentation for each detailed performance experiment;
receive result data of the amplification reaction performed by the nucleic acid detection device, using an amplification composition including the oligonucleotide prepared according to the preparation information for each detailed performance experiment;
process/analyze the received result data of the amplification reaction to obtain processed/analyzed data; and
store the data input via the data input screen for the item for experimentation, the preparation information, the result of the amplification reaction, and the processed/analyzed data as an experimental result documentation for each detailed performance experiment.

23. A computer program stored in a non-transitory computer-readable storage medium, wherein the computer program, when executed by at least one processor included in a computer device, causes the computer device to perform a method including:
providing a data input screen for at least one item for experimentation selected among items for experimentation, to be used in performing each detailed performance experiment on an oligonucleotide for detecting the target nucleic acid molecule, wherein the items include materials, experimental procedures, experimental devices, and a combination thereof;
displaying preparation information for a reaction plate, to be used for a nucleic acid detection device to perform an amplification reaction using the oligonucleotide, based on data input via the data input screen for the item for experimentation for each detailed performance experiment;
receiving result data of the amplification reaction performed by the nucleic acid detection device, using an amplification composition including the oligonucleotide prepared according to the preparation information for each detailed performance experiment;
processing/analyzing the received result data of the amplification reaction to obtain processed/analyzed data; and
storing the data input via the data input screen for the item for experimentation, the preparation information, the result of the amplification reaction, and the processed/analyzed data as an experimental result documentation for each detailed performance experiment.

24. A non-transitory computer-readable storage medium storing a computer program, wherein the computer program, when executed by at least one processor included in a computer device, causes the computer device to perform a method including:
providing a data input screen for at least one item for experimentation selected among items for experimentation, to be used in performing each detailed performance experiment on an oligonucleotide for detecting the target nucleic acid molecule, wherein the items include materials, experimental procedures, experimental devices, and a combination thereof;
displaying preparation information for a reaction plate, to be used for a nucleic acid detection device to perform an amplification reaction using the oligonucleotide, based on data input via the data input screen for the items for experimentation for each detailed performance experiment;
receiving result data of the amplification reaction performed by the nucleic acid detection device, using an amplification composition including the oligonucleotide prepared according to the preparation information for each detailed performance experiment;
processing/analyzing the received result data of the amplification reaction to obtain processed/analyzed data; and
storing the data input via the data input screen for the item for experimentation, the preparation information, the result of the amplification reaction, and the processed/analyzed data as an experimental result documentation for each detailed performance experiment.
